(19) Europäisches Patentamt / European Patent Office / Office européen des brevets

(11) **EP 3 675 723 B1**

(12) **EUROPEAN PATENT SPECIFICATION**

(45) Date of publication and mention
of the grant of the patent:
**19.11.2025 Bulletin 2025/47**

(21) Application number: **18851083.8**

(22) Date of filing: **08.08.2018**

(51) International Patent Classification (IPC):
*A61B 5/026* (2006.01)    *A61B 1/04* (2006.01)
*A61B 1/06* (2006.01)    *A61B 1/313* (2006.01)
*A61B 1/005* (2006.01)    *A61B 5/00* (2006.01)
*A61B 1/00* (2006.01)

(52) Cooperative Patent Classification (CPC):
**A61B 5/0075; A61B 1/000094; A61B 1/046;**
**A61B 1/063; A61B 1/0638; A61B 1/313;**
A61B 1/00163; A61B 1/043; A61B 5/0071;
A61B 5/0084; A61B 5/0261; A61B 2505/05

(86) International application number:
**PCT/US2018/045715**

(87) International publication number:
**WO 2019/045971 (07.03.2019 Gazette 2019/10)**

(54) **MULTI-SPECTRAL PHYSIOLOGIC VISUALIZATION (MSPV) USING LASER IMAGING METHODS AND SYSTEMS FOR BLOOD FLOW AND PERFUSION IMAGING AND QUANTIFICATION IN AN ENDOSCOPIC DESIGN**

MULTISPEKTRALE PHYSIOLOGISCHE VISUALISIERUNG (MSPV) UNTER VERWENDUNG VON LASERBILDGEBUNGSVERFAHREN UND -SYSTEMEN ZUR BLUTFLUSS- UND PERFUSIONSBILDGEBUNG UND -QUANTIFIZIERUNG IN EINEM ENDOSKOPISCHEN DESIGN

VISUALISATION PHYSIOLOGIQUE MULTI-SPECTRALE (MSPV) METTANT EN OEUVRE DES PROCÉDÉS ET DES SYSTÈMES D'IMAGERIE LASER POUR RÉALISER L'IMAGERIE ET LA QUANTIFICATION DE FLUX SANGUIN ET DE PERFUSION DANS UNE CONCEPTION ENDOSCOPIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2017 US 201715688472**

(43) Date of publication of application:
**08.07.2020 Bulletin 2020/28**

(60) Divisional application:
**25204421.9 / 4 646 994**

(73) Proprietor: **East Carolina University**
**Greenville, NC 27858 (US)**

(72) Inventors:
• **CHEN, Cheng**
**Greenville, North Carolina 27858 (US)**
• **FERGUSON, JR., T. Bruce**
**Raleigh, North Carolina 27615 (US)**
• **JACOBS, Kenneth Michael**
**Greenville, North Carolina 27834 (US)**

(74) Representative: **Boult Wade Tennant LLP**
**Salisbury Square House**
**8 Salisbury Square**
**London EC4Y 8AP (GB)**

(56) References cited:
WO-A1-2016/153741    JP-A- 2012 130 629
JP-A- 2015 223 463    US-A1- 2006 241 495
US-A1- 2008 132 794    US-A1- 2013 012 794
US-A1- 2014 187 966    US-A1- 2017 135 555

# EP 3 675 723 B1

## Description

### CLAIM OF PRIORITY

The present application claims the benefit of and priority to U.S. Application Publication No. US10390718B2, filed August 28, 2017.

### FIELD

The present inventive concept relates generally to blood flow and perfusion quantification and, more particularly, to quantification of blood flow and perfusion in terms of distribution of blood velocity and blood flow rate in tissue/organs using imaging techniques, such as Laser Speckle Imaging, Laser Doppler Imaging and the like with multispectral capability.

### BACKGROUND

The measurement results of blood flow and perfusion imaging technologies are typically disrupted by a motion artifact of the target tissue/organ in clinical circumstances. This movement can be micro (i.e., pulsatility of an arteriole due to systole and diastole blood pressure levels), intermediate (i.e., normal peristalsis of the small or large bowel) or macro (i.e., the movement of the heart during the cardiac cycle). This movement can be intrinsic to the imaged tissue (i.e., examples cited above), or extrinsic (i.e., the movement of the heart as a result of the movement of the lungs during ventilation). Thus, in many clinical situations, where accurate quantification of flow and perfusion is desirable, keeping the imaging target in a stationary status is difficult and, in some clinical scenarios, is not even possible. For example, such as imaging the distribution of blood flow velocity and flow rate for quantifying perfusion in coronary arteries and myocardium of a beating heart. Unfortunately, most conventional laser-based perfusion technologies either assume the target tissue/organ is stationary, which introduces significant inaccuracy or error in the clinical measurement of blood speed or velocity where the target is moving, such as a beating heart, or simply provide no information for quantification of perfusion in terms of blood flow rate distribution that is critically needed in the clinical situation where the target may or may not be moving.

Tissues/organs in animals or humans respond differently to light of different wavelengths. In general, light of shorter wavelengths can penetrate only the superficial layers of the tissues while light of longer wavelengths can penetrate both superficial layers and sub-surface layers in the spectral region from ultraviolet (UV) to near-infrared (NIR). UV and visible light of wavelengths less than, for example, 550nm is optimal for detailed anatomic visualization in medicine when viewing the surface of tissues and organs. However, unlike NIR light, UV or visible light imaging is usually not inherently capable of revealing the physiological characteristics of tissues/organs in sub-surface layers, in part due to lack of penetration of the tissues/organs. Accordingly, improved methods of visualization and quantification are desired.

US 2013/012794 A1 discloses methods and apparatus for video rate or near video rate quantitative imaging of tissue physiological and morphological properties from visible/NIR light spectral images obtain rapid multi-spectral reflectance images by illuminating with a series of spectra containing multiple narrow wavelength bands. An iterative light-transport based inversion algorithm may be applied for correcting the intensity of the spectral images from the geometry/coupling effect as well as from the scattering amplitude distortions. The method can produce video rate absorption as well as scattering spectral images that can be further analyzed very rapidly, using matrix-based rapid inversion algorithms to produce more detailed quantitative images containing information relevant to tissue physiology and morphology.

US 2006/241495 A1 discloses a polarization based diagnostic device for optically examining the medical condition of tissue comprises an illumination optical system comprising a light source and beam shaping optics. An optical detection system comprises imaging optics and an optical detector array, which detects light from the tissue. Polarizing optics provided in both the illumination optical system and the optical detection system are crossed and pass orthogonal polarization states. Iterative rotational means rotate the orthogonal polarization states relative to the tissue being examined. Image enhancement means includes image processing, sequential multi-spectral illumination and imaging, and image focus control to facilitate quality imaging at varying depths within the tissue. A controller operates the light source, the detector array, the multi-spectral illumination and imaging, image focus control, and image processing.

US 2008/132794 A1 discloses an apparatus for use in examining an object, such as skin, mucosa and cervical tissues for detecting cancer and precancerous conditions therein. In one embodiment, the apparatus includes a gun-shaped housing having a handle portion and a barrel portion. The front end of the barrel portion is open, and a glass cover is mounted therein. LED's are disposed within the handle portion. A manually-operable switch for controlling actuation of the LED's is accessible on the handle portion. An optical fiber is used to transmit light from the LED's through a first polarizer in the barrel portion and then through the glass cover to illuminate a desired object. Reflected light from the object is passed through a second polarizer, which is adjustably mounted in the barrel portion and which is preferably oriented to pass depolarized light emitted from an illuminated object, and is then imaged by optics onto a CCD detector. The detector is coupled to a wireless transmitter that transmits the output from the detector to a remotely located wireless receiver.

WO 2016/153741 A1 discloses a system that uses two wavelengths of differential transmittance through a sample to apply laser speckle or laser Doppler imaging. A first of the two wavelengths is within the visible range that has zero or very shallow penetration. This wavelength captures the anatomical structure of tissue/organ surface and serves as a position marker of the sample but not the subsurface movement of blood flow and perfusion. A second wavelength is in the near Infra-Red (NIR) range, which has much deeper penetration. This wavelength reveals the underlying blood flow physiology and correlates both to the motion of the sample and also the movement of blood flow and perfusion. Thus, true motion of blood flow and perfusion can be derived from the NIR imaging measurement without being affected by the motion artifact of the target.

US 2017/135555 A1 discloses a n image processing method includes: acquiring correction data for correcting first image data into second image data, the first image data being generated by an imaging device when a subject is irradiated with three rays of narrow band light having wavelength bands narrower than those of spectral sensitivity of pixels R, G, and B, and having spectrum peaks within wavelength bands of the spectral sensitivity of the pixels R, G, and B, and the second image data being deemed to be generated by the imaging device when white light is emitted; acquiring the first image data when the subject is irradiated with the three rays of narrow band light; generating color image data of the second image data using the first image data and the correction data; and calculating oxygen saturation of the subject using a pixel value R and a pixel value G included in the first image data.

## SUMMARY OF THE INVENTION

The present invention is defined in the appended claims. According to a first aspect of the present invention there is provided a multispectral imaging system as defined in claim 1. According to a second aspect of the present invention there is provided non invasive method for multispectral imaging in a multispectral imaging system, excluding endoscopic procedures that include manipulation of tissue as defined in claim 13. Further aspects of the present invention are defined in the dependent claims.

## SUMMARY OF THE DISCLOSURE

Some embodiments provide a multispectral imaging system. The system includes a first light source having a first wavelength configured to image a sample; a second light source, different from the first light source, having a second wavelength, different from the first wavelength, configured to image the sample; at least a third light source, different from the first and second light sources, having a third wavelength, different from the first and second wavelengths, configured to image the sample; a camera configured to receive information related to the first, second and at least third light sources from the sample, wherein light at the first wavelength is configured to image a surface of the sample into the camera; light at the second wavelength is configured to penetrate the sample to a first depth and provide information related to the sample to the camera; and light at least the third wavelength is configured to penetrate the sample to a second depth different from the first depth of the light at the second wavelength; and a processor configured to combine the information related to the first, second and at least third light sources provided by the camera to image an anatomical structure of the sample, image physiology of blood flow and perfusion of the sample and/or synthesize the anatomical structure and the physiology of blood flow and perfusion of the sample in terms of a blood flow rate distribution. The camera is positioned in a region of interest of the sample using an endoscope.

In further embodiments, the endoscope may be configured to provide the information related to the first, second and at least third light sources to the processor and may be included in an endoscopic imaging system. The endoscopic imaging system may include the camera; a control unit configured to control the camera and process images associated therewith; an image management unit configured to display and manipulate the images associated with the camera; and a system interface configured to translate the information related to the first, second and at least third light source to perform within the endoscopic imaging system such that the images associated therewith are displayed and manipulated.

In still further embodiments, the endoscopic imaging system may include at least one of a system for endoscopic surgery including one or more of laparoscopy, thorascoscopy, and cystoscopy; a system for minimally invasive surgical procedures using the endoscope for illumination, visualizing, and manipulation; and a system for robotic procedures using the endoscope for illumination, visualizing, and manipulation.

In some embodiments, the endoscopic imaging system may be configured to illuminate cavity tissues and organs during a procedure; to visualize cavity tissues and organs for surgical intervention; and for surgical manipulation of cavity tissues and organs for surgical intervention.

In further embodiments, the endoscopic system may be configured to illuminate the region of interest using fibers, fiber compositions, fiber arrangements, lenses, diffusers, collimators, and/or expanders. In still further embodiments, the camera may include a plurality sensors and is configured to have speeds, focus, accuracy and fidelity to obtain images using the endoscopic system. The camera may be coupled to the optic fiber in the endoscope.

In some embodiments, the plurality sensors may be arranged along a continuum from an endoscope tip to a distance remote from the endoscope coupled by image capture cabling.

In further embodiments, the endoscope may be one of a rigid endoscope, semi-rigid endoscope and a flexible endoscope.

In still further embodiments, the endoscope may be one of a rigid endoscope having a flexible, navigable tip and a flexible endoscope having a flexible insertion tube.

In some embodiments, the at least third wavelength may be configured to assess a specific physiologic parameter, such as Hgb concentration. The system may be provided as one or more of a hand-held system, a finger probe unit, a skin patch, and a mobile system.

In further embodiments, the multispectral imaging system may include different blood flow and perfusion measurement technologies in one system. The blood flow and perfusion measurement technologies may include one or more of laser speckle imaging (LSI), laser Doppler imaging (LDI), florescence imaging and reflectance imaging.

Still further embodiments provide methods for multispectral imaging in a multispectral imaging system adapted for an endoscopic system. The method includes imaging a sample using a first light source having a first wavelength and being delivered through an endoscopic system; imaging the sample using a second light source, different from the first light source, having a second wavelength, different from the first wavelength, and being delivered through the endoscopic system; receiving information related to the first and second light sources from the sample at a camera, wherein light at the first wavelength is configured to reflect off a surface of the sample into the camera and light at the second wavelength is configured to penetrate the sample and provide information related to the sample to the camera through the endoscopic system; and combining the information related to the first and second light sources received by the camera using at least one processor to image an anatomical structure of the sample, image physiology of blood flow and perfusion of the sample and/or synthesize the anatomical structure and the physiology of blood flow and perfusion of the sample in terms of a blood flow rate distribution

In some embodiments, the illumination, analysis and display may be performed using the endoscopic system. The endoscopic system may be configured to communicate with a multi-spectral physiologic visualization (MSPV) operating system.

Some embodiments provide an endoscopic imaging system. The system includes an endoscope and a light source unit coupled to the endoscope. The light source unit provides first, second and at least a third light source. The first light source has a first wavelength configured to image a sample. The second light source is different from the first light source and has a second wavelength, different from the first wavelength, configured to image the sample. The at least a third light source, different from the first and second light sources, has a third wavelength, different from the first and second wavelengths, configured to image the sample. The system further includes a camera control unit coupled to the endoscope through a camera head of a camera, the camera head being adapted to receive information related to the first, second and at least third light sources, wherein light at the first wavelength is configured to image a surface of the sample into the camera; light at the second wavelength is configured to penetrate the sample to a first depth and provide information related to the sample to the camera and light at the at least third wavelength is configured to penetrate the sample to a second depth and provide information related to the sample to the camera; and an image processing unit coupled to endoscope configured to combine the information related to the first, second and at least third light sources provided by the camera head to image an anatomical structure of the sample, image physiology of blood flow and perfusion of the sample and/or synthesize the anatomical structure and the physiology of blood flow and perfusion of the sample in terms of a blood flow rate distribution.

**BRIEF DESCRIPTION OF THE DRAWINGS**

Figure 1A is a block diagram illustrating a system implementing dual wavelength imaging in accordance with some embodiments.

Figure 1B is a block diagram illustrating a system including multiple light sources in accordance with some embodiments.

Figure 1C is a block diagram illustrating a multi-sensor camera in accordance with some embodiments.

Figure 2 is a more detailed block diagram illustrating various components of a multi-wavelength imaging system in accordance with some embodiments.

Figure 3 is a block diagram of a data processing system according to some embodiments.

Figure 4 is a more detailed block diagram of the data processing system illustrated in Figure 3 in accordance with some embodiments.

Figures 5A and 5B are a visible light image (5A) and a near infra-red light image (5B) of a hand.

Figures 6A and 6B are images illustrating the perfusion measurement using only near infra-red light (6A) and dual wavelength illumination (6B) of a stationary hand.

Figures 7A and 7B are images illustrating the perfusion measurement using only near infra-red light (7A) and dual wavelength illumination 7B) of a moving hand.

Figures 8A and 8B are images illustrating the perfusion measurement using only near infra-red light (8A) and dual wavelength illumination (8B) of a stationary hand with blood supply temporarily occluded by inflating an ipsilateral blood pressure cuff.

Figures 9A and 9B illustrated perfusion measurement using only near infra-red light (9A) and dual wavelength illumination (9B) of a large bowel of a pig.

Figures 10A-10D are images illustrating a visible light image of a piece of small bowel of a pig as to define anatomical structure (10A); a near infra-red light image of the same piece of small bowel as to define the transparency map (10B); blood flow speed distribution map of the same piece of small bowel calculated by 11 frames of the NIR raw images using LSI (10C); and a combined visual effect using A, B, C using an algorithm in accordance with some embodiments to reveal both anatomical structure and blood flow physiology (10D).

Figures 11A-11C are images illustrating a visible light image of a piece of small bowel of a pig as to define anatomical structure by the brightness of the 8 bit grayscale image (11A); blood flow speed distribution map of the same piece of small bowel calculated by 11 frames of the NIR raw images using LSI (11B); and a combined visual effect using A and B using an algorithm in accordance with some embodiments to reveal both anatomical structure and blood flow physiology (11C).

Figures 12A-12D are images illustrating Panel A, an NIR 785nm image of a small bowel (12A); Panel B a Green 532nm image of the same small bowel (12B); Panel C, a reconstructed image of the same small bowel (12C); and Panel D, an image of the same small bowel taken by a regular camera (12D).

Figures 13A-13D are images illustrating Panel A, an NIR 785nm image of a pig heart (13A); Panel B, Green 532nm image of the same pig heart (13B); Panel C, a reconstructed image of the same pig heart (13C); and Panel D, an image of the same pig heart taken by a regular camera (13D).

Figures 14A-14E illustrate an image using a visible wavelength (532nm) (14A); an image using near infra-red wavelength (785nm) (14B); a reconstructed image (in gray scale) with the visible and infrared wavelengths (14C); a regular image with room light illumination (14D); and an image showing blood flow and perfusion image (14E).

Figures 15A-15B, 16A-16B, 17A-17B, 18A-18B, and 19A-19B illustrate images that compensate for issues during clinical imaging procedures in accordance with some embodiments.

Figure 20 is a diagram of a mobile system in accordance with some embodiments.

Figures 21A and 21B are diagrams illustrating a smaller footprint system in accordance with some embodiments.

Figure 22 is a diagram illustrating a finger probe device and/or skin patch in accordance with some embodiments.

Figure 23 is a side perspective view of a handheld mobile system in accordance with some embodiments.

Figure 24 is a diagram of a standalone system including an endoscopic device in accordance with some embodiments.

Figure 25 is a block diagram of an adaptive system in accordance with some embodiments.

Figures 26A through 26D are images of a human hand captured in accordance with embodiments.

Figure 27 is a block diagram of a system including an endoscope in accordance with various embodiments.

Figure 28 is line drawing of an example system of Figure 27 in accordance with some embodiments.

Figures 29A and 29B illustrate images and an image quality indicator bar used in a quality check method in accordance with some embodiments.

Figure 30 is a flowchart illustrating methods in accordance with some embodiments.

Figure 31 is a diagram illustrating field of view (FOV) in accordance with endoscopic embodiments discussed herein.

## DETAILED DESCRIPTION

Embodiments of the present inventive concept will now be described more fully hereinafter with reference to the accompanying figures, in which preferred embodiments of the inventive concept are shown. This inventive concept may, however, be embodied in many different forms and should not be construed as limited to the embodiments set forth herein. Like numbers refer to like elements throughout. In the figures, layers, regions, elements or components may be exaggerated for clarity. Broken lines illustrate optional features or operations unless specified otherwise.

The terminology used herein is for the purpose of describing particular embodiments only and is not intended to be limiting of the inventive concept. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y." The term "about" means the numerical value can vary by plus or minus ten percent.

Unless otherwise defined, all terms (including technical and scientific terms) used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

It will be understood that, although the terms first, second, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the inventive concept. The sequence of operations (or steps) is not limited to the order presented in the claims or figures unless specifically indicated otherwise.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

As will be appreciated by one of skill in the art, embodiments of the present inventive concept may be embodied as a method, system, data processing system, or computer program product. Accordingly, the present inventive concept may take the form of an embodiment combining software and hardware aspects, all generally referred to herein as a "circuit" or "module." Furthermore, the present inventive concept may take the form of a computer program product on a non-transitory computer usable storage medium having computer usable program code embodied in the medium. Any suitable computer readable medium may be utilized including hard disks, CD ROMs, optical storage devices, or other electronic storage devices.

Computer program code for carrying out operations of the present inventive concept may be written in an object oriented programming language such as Matlab, Mathematica, Java, Smalltalk, C or C++. However, the computer program code for carrying out operations of the present inventive concept may also be written in conventional procedural programming languages, such as the "C" programming language or in a visually oriented programming environment, such as Visual Basic.

It will be understood that some embodiments of the present inventive concept implemented in Matlab may provide improved processing speeds in accordance with some embodiments of the present inventive concept.

Certain of the program code may execute entirely on one or more of a user's computer, partly on the user's computer, as a standalone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

The inventive concept is described in part below with reference to flowchart illustrations and/or block diagrams of methods, devices, systems, computer program products and data and/or system architecture structures according to embodiments of the inventive concept. It will be understood that each block of the illustrations, and/or combinations of blocks, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the block or blocks.

These computer program instructions may also be stored in a computer readable memory or storage that can direct a computer or other programmable data processing apparatus to function in a particular manner, such that the instructions stored in the computer-readable memory or storage produce an article of manufacture including instruction means which implement the function/act specified in the block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the block or blocks.

The present inventive concept relates generally to blood flow and perfusion quantification and, more particularly, to quantification of blood flow and perfusion in tissue/organs in terms of distributions of blood velocity and blood flow rate using imaging techniques, such as Laser Speckle Imaging (LSI), Laser Doppler Imaging (LDI), Florescence imaging, reflectance imaging and the like with multispectral capability. Some embodiments of the inventive concept use two or more wavelengths in the range from 350 nm to 1100 nm to measure/quantify the blood velocity and blood flow rate distributions for quantification of perfusion, remove motion artifact and enhance visualization for presentation and real-time evaluation and assessment of the synthesized anatomical-physiological result. As used here, "Multispectral Laser Imaging (MSLI)" refers to imaging techniques using two or more wavelengths in accordance with some embodiments of the present inventive concept.

In particular, some embodiments of the present inventive concept provide a system that uses two wavelengths (or wavelength ranges) of differential transmittance through a sample to apply laser speckle or laser Doppler imaging. A first of the two wavelengths may be relatively small within the UV or visible range, such as blue light 450-495 nm. Light at this wavelength has very shallow penetration and images the anatomical structure of tissue/organ surface and serves as a position marker of the sample but not the subsurface movement of blood flow and perfusion. A second wavelength may be relatively large in the visible (400-700 nm) or near Infra-Red (NIR) range (700-2500 nm). Light at this wavelength has much larger penetration depth and reveals the underlying blood flow physiology and correlates both to the motion of the sample and also the movement of blood flow and perfusion. Using the imaging measurement of the visible light as a baseline, the true motion of blood flow and perfusion can be derived from the NIR imaging measurement without being affected by the motion artifact of the target. Furthermore, the anatomical structure information captured by visible light and the physiological characteristics measured by NIR light is combined as will be discussed herein.

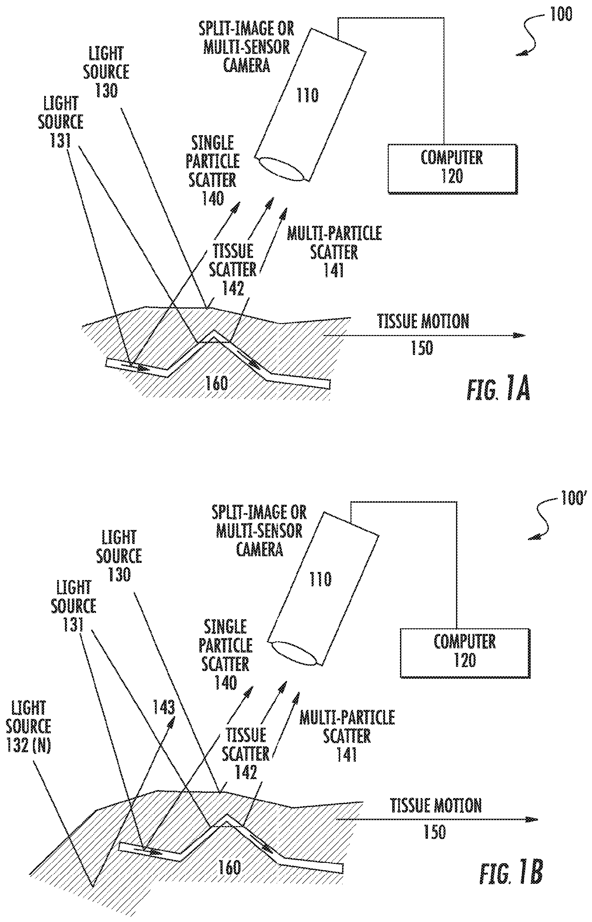

As discussed in the background of the present application, using only visible or NIR spectrums may result in various issues with the final images produced. Accordingly, some embodiments of the present inventive concept combine different wavelengths of visible and NIR spectrum (350 nm - 1100 nm) into an imaging system, such as LSI, LDI, Fluorescence, Reflectance or LSI plus Fluorescence and the like. The combination, as discussed herein, may reveal much more information of the tissue/organ than using one single wavelength. In particular, MSLI in accordance with some embodiments discussed herein can (1) account for and remove the motion artifact present in imaging clinical biologic structures, which creates blood flow and perfusion quantification inaccuracies; (2) improve visualization over current technologies by exact synthesis of both anatomic structure and the physiology of blood flow and perfusion simultaneously in real time; (3) through a combination of (1) and (2), improve the accuracy of quantification of blood flow and perfusion in clinical applications as will be discussed herein with respect to Figures 1A through 29B.

As used herein, "real time" refers to provision of data within a very short amount of time, for example, milliseconds, so as to appear as if the data was provided immediately upon request or activation of light sources.

In some embodiments, in addition to using multiple wavelengths over the visible and NIR spectrum (350-1100 nm), embodiments of the present inventive concept can, for example, combine two or more laser imaging techniques such as near infra-red fluorescence (NIRF) and Laser Speckle Imaging (LSI), or NIRF and Laser Doppler Imaging (LDI), into one system as will also be discussed below with respect to the Figures.

Furthermore, some embodiments of the present inventive concept provide the ability to apply methods of visualization and quantification across multiple clinical and experimental settings. These settings include direct illumination and imaging of tissues, but where access to the imaged Field of View (FOV) is accomplished through different approaches. These approaches may include, for example, direct contact or non-contact with tissues, exposure of the tissues during open surgical procedures, or via endoscopy to access tissues within closed anatomic structures or tissues in the alimentary tract or tracheobronchial tree without departing from the scope of the present inventive concept.

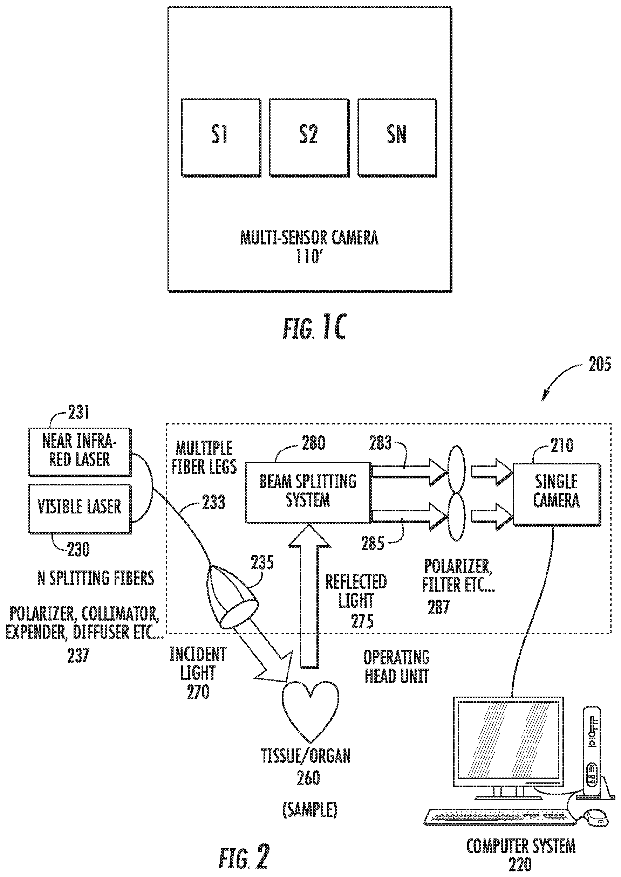

Referring first to Figure 1A, a block diagram illustrating a simplistic system implementing dual wavelength imaging in accordance with some embodiments of the present inventive concept will be discussed. As illustrated in Figure 1A, the system 100 includes at least two light sources, first 130 and second 131 light sources, respectively, a sample 160, a camera 110 and a communications device (computer 120). In some embodiments of the present inventive concept, the first light source 130 delivers visible light and the second light source 131 delivers NIR light. As discussed above, the coherent short wavelength (visible source 130) does not penetrate deep into the sample 160 (tissue/organ), but provides detail of the surface of the sample 160 in the tissue scatter (142). In contrast, the coherent NIR source 131 penetrates deep into the sample 160 and may provide single (140) or multi particle (141) scatter. The reflections 140, 141, 142 off the sample 160 are captured by a camera 110, which may be, for example, a split-image or multi-sensor camera. In particular, in some embodiments the camera may be a multi-sensor camera, rather than a single camera with one sensor chip. The multi-sensor camera has multiple sensors and each sensor may be configured to image one wavelength or wavelength range. As illustrated in Figure 1C, in embodiments having a multi-sensor camera 110', the camera may have a plurality of spaced apart sensors S1 through SN. Each sensor may be configured to image one wavelength or wavelength range. The number "N" in S1-SN can be any reasonable number in accordance with embodiments discussed here. For example, "N" may be between 2 and 50.

The information can be processed by the communications device 120, which combines the visible and NIR wavelength images to provide improved blood flow and profusion data in accordance with some embodiments of the present inventive concept. As will be understood, the data provided by embodiments discussed herein account for movement 150 of the sample (tissue/organ) 160 and provide a much improved image thereof.

Although some embodiments are discussed herein as having two wavelengths, embodiments of the present inventive concept are not limited to this configuration. For example, as illustrated in the system 100' in Figure 1B, in some embodiments, at least a third light source 132 is provided having a third wavelength and this wavelength may penetrate the sample a different depth than the first and second wavelengths and provide a different scatter pattern 143. In some embodiments, the at least third wavelength may be configured to assess a specific physiologic parameter, for example, Hgb concentration. It will be understood that there may more than three light sources in some embodiments.

Referring now to Figure 2, a more detailed block diagram illustrating various components of a multi-wavelength imaging system in accordance with some embodiments of the present inventive concept will be discussed. As illustrated in Figure 2, the system 205 includes at least two laser light sources, visible 230 and NIR 231, a connecting fiber 233, components of an imaging system 237, a sample 260, a beam splitter 280, a camera 210 and a communications device (computer system 220). In operation, when the NIR laser delivers NIR light to a living sample 260, such as a tissue/organ, a portion of the NIR light will go through single or multiple scattering of both stationary and moving particles inside the sample and reflect back. When the visible laser 230 delivers non-penetrating visible light, such as light having 430nm, to a living sample 260, such as a tissue/organ, most of the light will be reflected back by the surface within less than $100\mu m$ depth. For the NIR laser 231, approximately ninety five percent of the light will be returned from a top $700\mu m$ of the sample 260, which is enough penetration to pass through coronary artery walls at, for example, a $300\mu m$ depth, and generate information from moving particles, such as red blood cells, as well as from stationary tissue.

The reflected visible light contains the surface movement information of the sample 260 and, thus, reflects the motion artifact. The reflected NIR light contains the surface and subsurface movement information of the sample 260 and, thus, reflects both motion artifact and movement of the blood flow. As illustrated in Figure 2, the light produced by the lasers 230 and 231 may be provided to a fiber 233, which may have multiple fiber legs and may include a plurality of splitting fibers 235 as illustrated. However, embodiments of the present inventive concept are not limited to the configuration illustrated in Figure 2. For example, more or less fibers may be used without departing from a scope of the present inventive concept. Furthermore, the light on the fibers may pass through various elements of an imaging system 237 before reaching the sample 260. For example, the light may traverse polarizers, collimators, expanders, diffusers and the like before reaching the sample 260 without departing from the scope of the present inventive concept.

The incident light 270 illuminates the sample 260 and the reflected light 275 is provided to a beamsplitter 280. In some embodiments of the present inventive concept, the beamsplitter 280 may be a dichroic beam splitting system that separates the NIR 283 and visible light 285. The separated light 283 and 285 may pass through polarizers, filters and the like 287 before being delivered to the camera 210. As discussed above, the camera 210 can be, for example, a split-image (single sensor) or multi-sensor camera without departing from the scope of the present inventive concept. As stated, the multi-sensor camera has multiple sensors each configured to image a wavelength or wavelength range.

The NIR 283 and visible 285 images are directed to the camera 210 and a split image is created on one camera sensor or on separate camera sensors S1-SN (Figure 1C) that have been synchronized and aligned. As discussed above, different wavelengths have different penetration levels in the tissue/organ. Using multi-spectrum image design as discussed herein, the anatomical structure and blood flow physiology at different depths in the tissue/organ can be revealed as will be discussed below with respect to various figures.

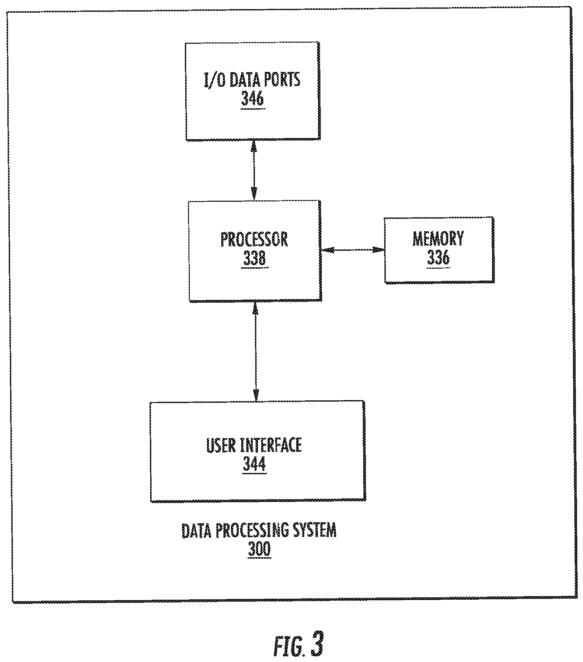

As illustrated in Figures 1A, 1B and 2, systems in accordance with embodiments of the present inventive concept include communications devices 120, 220, which are used for the various processing necessary to implement embodiments of the present inventive concept. Referring now to Figure 3, a data processing system 300 that may be used in the systems of Figures 1 and 2, for example, in the communications devices 120, 210, in accordance with some embodiments of the inventive concept will be discussed. It will be understood that the data processing system 300 may be included in any of the components of the system without departing from the scope of the present inventive concept. For example, the data processing system 300 may be included in the camera 110, 210 or split between various elements of the system without departing from the scope of the present inventive concept.

Referring now to Figure 3, an exemplary embodiment of a data processing system 300 suitable for use in the systems of Figures 1 and 2 includes a user interface 344 such as a keyboard, keypad, touchpad or the like, I/O data ports 346 and a memory 336 that communicates with a processor 338. The I/O data ports 346 can be used to transfer information between the data processing system 300 and another computer system or a network. These components may be conventional components, such as those used in many conventional data processing systems, which may be configured to operate as described herein.

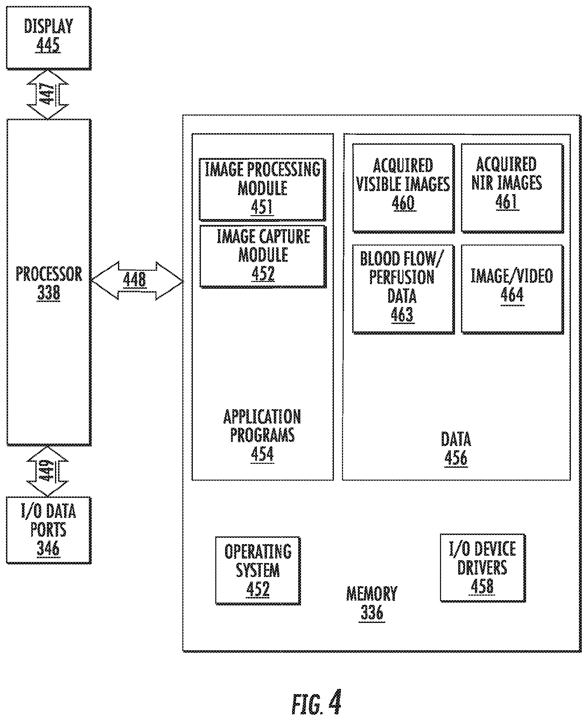

Referring now to Figure 4, a more detailed block diagram of the data processing system 400 in accordance with some embodiments of the present inventive concept will be discussed. The processor 338 communicates with a display 445 via and address/data bus 447, the memory 336 via an address/data bus 448 and the I/O data ports 346 via an address/date bus 449. The processor 338 can be any commercially available or custom microprocessor or ASICs. The memory 336 is representative of the overall hierarchy of memory devices containing the software and data used to implement the functionality of the data processing system 400. The memory 336 can include, but is not limited to, the following types of devices: cache, ROM, PROM, EPROM, EEPROM, flash memory, SRAM, and DRAM.

As illustrated in Figure 4, the memory 336 may include several categories of software and data used in the data processing system 400: an operating system 452; application programs 454; input/output (I/O) device drivers 458; and data 456. As will be appreciated by those of skill in the art, the operating system 452 may be any operating system suitable for use with a data processing system, such as OS/2, AIX or zOS from International Business Machines Corporation, Armonk, NY, Windows95, Windows98, Windows2000, WindowsXP, or Vista from Microsoft Corporation, Redmond, WA, Unix, Linux, LabView, or a real-time operating system such as QNX or VxWorks, or the like. The I/O device drivers 458 typically include software routines accessed through the operating system 452 by the application programs 454 to communicate with devices such as the I/O data port(s) 346 and certain memory 336 components. The application programs 454 are illustrative of the programs that implement the various features of the data processing system 400 included in a system in accordance with some embodiments of the present inventive concept and preferably include at least one application that supports operations according to some embodiments of the present inventive concept. Finally, the data 456 represents the static and dynamic data used by the application programs 454, the operating system 452, the I/O device drivers 458, and other software programs that may reside in the memory 336.

As illustrated in Figure 4, the data 456 according to some embodiments of the present inventive concept may include acquired visible images 460, acquired NIR images/data 461, calculated blood flow/perfusion data 463 and images/video 464. Although the data 456 illustrated in Figure 4 includes four different files 460, 461, 463 and 464, embodiments of the present inventive concept are not limited to this configuration. Two or more files may be combined to make a single file; a single file may be split into two or more files and the like without departing from the scope of the present inventive concept.

As further illustrated in Figure 4, the application programs 454 may include an image processing module 451 and an image capture module 452 in accordance with some embodiments of the inventive concept. While the present inventive concept is illustrated, for example, with reference to the image processing module 451 and the image capture module 452 being application programs in Figure 4, as will be appreciated by those of skill in the art, other configurations may also be utilized while still benefiting from the teachings of the present inventive concept. For example, the image processing module 451 and the image capture module 452 may also be incorporated into the operating system 452 or other such logical division of the data processing system 400. Thus, the present inventive concept should not be construed as limited to the configuration of Figure 4, but is intended to encompass any configuration capable of carrying out the operations described herein.

Furthermore, while the image processing module 451 and the image capture module 452 are illustrated in a single data processing system, as will be appreciated by those of skill in the art, such functionality may be distributed across one or more data processing systems. Thus, the present inventive concept should not be construed as limited to the configurations illustrated in Figures 3 and 4, but may be provided by other arrangements and/or divisions of function between data processing systems.

In certain embodiments, such as an LSI application, the velocity of a target fluid can be calculated using the following equation:

$$v(i, j) = v_0 + \frac{a}{c(i,j)^2} \qquad \text{Eqn. (1)}$$

where $v(i, j)$ is the velocity of target fluid, $v_0 K_{LSCI}(x, y)_0$ is an added term to account for background noise and may be zero after the baseline has been removed; a is a constant related to imaging parameters, laser parameters, time/spatial smoothing parameters for obtaining c and reflects the optical characteristics of the target fluid; c is the laser speckle contrast; and i and j are the row and column pixel index.

For an LDI application, the velocity of a target fluid can be calculated using the following equation:

$$v(i, j) = \frac{\lambda}{2 \sin \theta} \Delta f \qquad \text{Eqn. (2)}$$

where $v(i, j)$ is velocity of target fluid; where $\lambda$ is the wavelength; $\Delta f$ is the change in Doppler frequency (Doppler frequency shift); and $\theta$ is half of the angle between the two beams. Typically, there is no direct formula to apply for NIRF, and the like.

However, even when the imaged object is stationary, there is movement present that must be accounted for to accurately determine blood flow in vessels and perfusion in tissue. As recently as 2013, experts in the field of LSI discussed motion artifact as one of the two key questions still to be answered in this field. Therefore, systems and methods that have the capability to identify this motion contribution and account for its magnitude are needed and included in technologies claiming to be able to assess, image, and /or quantify blood flow in vessels and perfusion in tissues experimentally and in vivo.

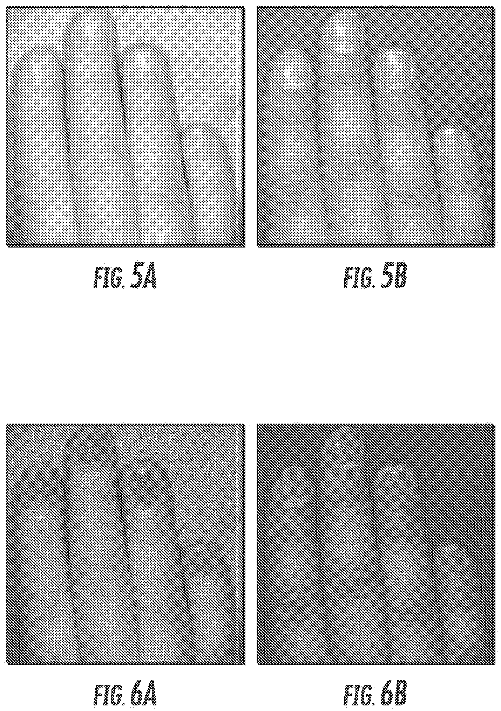

Referring now to Figures 5A and 5B, Figure 5A is a visible light image of a hand and Figure 5B is a near infra-red light image of a hand. These images may be used to calculate the motion artifact and the movement of the blood flow and perfusion in accordance with some embodiments of the present inventive concept.

In particular, to remove the motion artifact of the tissue/organ that is caused by movement of tissue/organ, such as aspiration, spasm, heart beat and the like and/or the camera, Galilean velocity addition can be calculated using the following equation:

$$\mathbf{v}_{12}(\mathbf{r}) = \mathbf{v}_{13}(\mathbf{r}) + \mathbf{v}_{32}(\mathbf{r}) = \mathbf{v}_{13}(\mathbf{r}) - \mathbf{v}_{23}(\mathbf{r}) \qquad \text{Eqn. (3)}$$

where: $\mathbf{v}_{13}(\mathbf{r})$ is the velocity distribution of object of interest (blood flow and perfusion) relative to detector (camera); $v_{23}(r)$ is the velocity distribution of the host object (the tissue/organ in which the blood vessel is embedded) relative to detector (camera); $\mathbf{v}_{32}(\mathbf{r})$ is the velocity distribution of the detector (camera) relative to the host object (the tissue/organ in which the blood is embedded); and $v_{12}(r)$ is the velocity distribution of an object of interest (blood flow and perfusion) relative to the host object (the tissue/organ in which the blood vessel is embedded). In other words, $\mathbf{v}_{32}(\mathbf{r})$ and $v_{23}(r)$ Thus, embodiments of the present inventive concept may address a need to determine $v_{12}(r)$ under the condition that the image signals by the all the current LSI or LDI method provides only $\mathbf{v}_{13}(\mathbf{r})$. According to some embodiments of the present inventive concept, the multi spectrum imaging approach, both $v_{13}(r)$ and $v_{23}(r)$ can be made available.

Using LSI as an example, using the Eqn. (1) above, the speckle contrast of coherent NIR laser light $C_{NIR}(i, j)$ is

associated with $v_{13}(\mathbf{r})$, which is the velocity distribution of an object of interest (blood flow and perfusion) relative to detector (camera). $v_{13}(r)$ is affected by the movement of blood flow and the movement of tissue/organ caused by factors such as aspiration, spasm, heart beat etc. and the movement of the camera. The visible laser light, especially within the 450~495nm wavelength range (blue laser light), has much less penetration in soft tissue/organ compared with the NIR laser light.

Using Eqn. (1) set out above, the speckle contrast of coherent visible laser light $C_{VIS}(i, j)$ is mainly associated with $v_{23}(\mathbf{r})$, which is the velocity distribution of the host object (the tissue/organ that the blood vessel is embed) relative to detector (camera). $v_{23}(\mathbf{r})$ is affected by the movement of tissue/organ caused by factors such as aspiration, spasm, heart beat etc. and the movement of the camera. Using Eqn. (3), $v_{12}(\mathbf{r})$ can be derived using $v_{13}(\mathbf{r})$ and $v_{23}(\mathbf{r})$ thus the velocity distribution of object of interest (blood flow and perfusion) relative to the host object (the tissue/organ that the blood vessel is embed) can be quantified without the effect of the movement of tissue/organ and the movement of the camera.

The speckle contrast of coherent visible laser light $C_{VIS}(i, j)$ as a baseline can be used to normalize the speckle contrast of coherent NIR laser light $C_{NIR}(i, j)$ based on this mathematic model to reduce the velocity component of the motion artifact. Computer algorithms may be designed to normalize (subtract or divide) $C_{NIR}(i, j)$ using $C_{VIS}(i, j)$ to yield one or multiple stabilized blood flow and perfusion maps in real time. The algorithms may be processed by, for example, a data processor as discussed above with respect to Figures 3-4.

Referring now to Figures 6A and 6B, images generated using the measurement of the blood flow and perfusion using only NIR and dual wavelength illumination of a stationary hand will be discussed. As illustrated, the measurement of the blood flow and perfusion using only NIR and dual wavelength illumination of a stationary hand are very similar. This is because when the sample/target is stationary, the motion artifact as baseline measured by visible light is close to zero. Thus, the result without removing the baseline (Figure 6A: using only NIR light) and the result with the baseline removed (Figure 6B: using dual wavelength illumination) are almost identical.

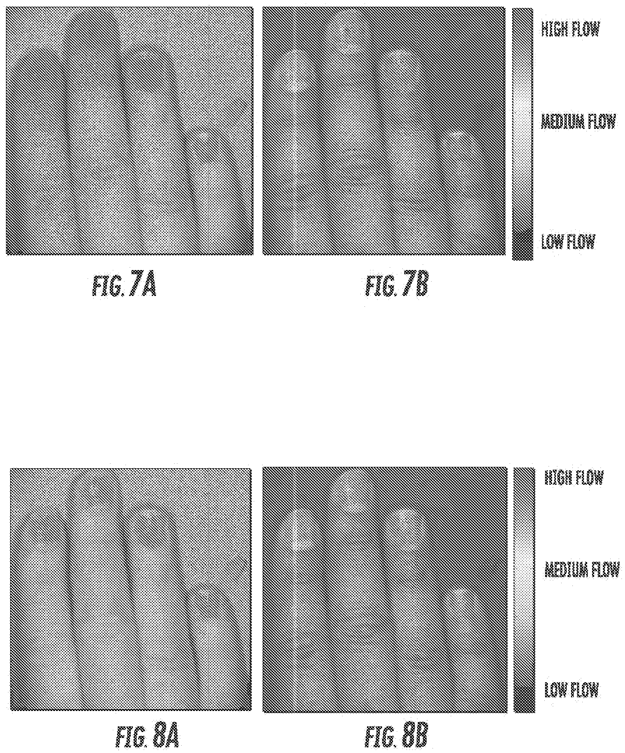

Referring now to Figures 7A and 7B, images illustrating the measurement of the blood flow and perfusion using only NIR and dual wavelength illumination of a moving hand will be discussed. As illustrated therein, the measurement of the blood flow and perfusion using only NIR and dual wavelength illumination of a shaking hand are very different. The measurement with only NIR light (Figure 7A) shows much higher perfusion level which is caused by the motion artifact. The measurement with dual wavelength illumination (Figure 7B) is almost identical to the measurement of the stationary hand. This is because when the sample/target is moving the motion artifact as baseline measured by visible light is not zero. Thus, the result without removing the baseline (Figure 7A: using only NIR light) shows more "blood flow and perfusion" than the result with the baseline removed (Figure 7B: using dual wavelength illumination).

Referring now to Figures 8A and 8B, images illustrating both the perfusion measurement with only NIR and the dual wavelength illumination will be discussed. In particular, Figures 8A and 8B are images illustrating the perfusion measurement using only near infra-red light (8A) and dual wavelength illumination (8B) of a stationary hand with blood supply temporarily occluded by squeezing the wrist of the imaged hand using the other hand. As illustrated, a decrease induced by the temporary occlusion of the blood supply to the hand is clear.

Different from LSI, LDI uses interference of two coherent light beams: the one from the laser as the light source and the one reflected from the moving object whose frequency is slightly shifted from that of the incident light. LDI determines the speed of one "pixel" or points or a small region of the object where the incident beam is focused on. An image is obtained by scanning the focused beam. Similar to the LSI of Eqn. (1) using Eqn. (2), measurement of $v_{13}(r)$ and $v_{23}(r)$ in LDI can be achieved using a penetrating NIR beam and a non-penetrating visible beam. Again, using Eqn. (3) $v_{12}(r)$ of the fiducial points relative to the host object (the tissue/organ that the blood vessel is embed) can be identified.

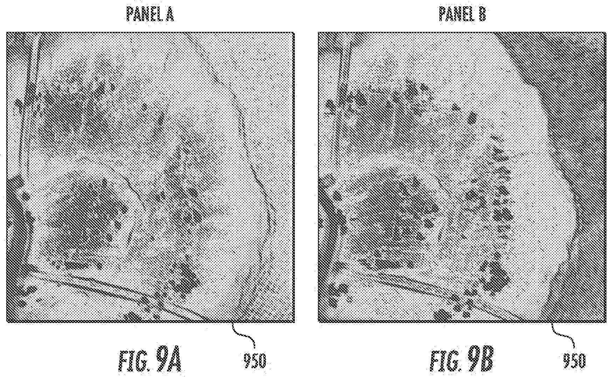

Furthermore, practically, the laser speckle contrast is a mixture of static background and dynamic part. The dynamic part of the speckle contrast is associated with the motion and the static background is caused by the difference of the optical characteristics of the inhomogeneous scattering media. Since among the current LSI technologies, baseline speckle contrast at a no flow situation is not available, other than in a controlled phantom/tubing experiment, the static background of the speckle contrast is a major obstacle to accurately quantifying blood flow in tissue/organ. Multi-spectrum illumination schemes provide a baseline speckle contrast at no flow situation $C_{VIS}(i, j)$ using visible coherent laser light. The speckle contrast of coherent visible laser light $C_{VIS}(i, j)$ can be used to normalize the speckle contrast of coherent NIR laser light $C_{NIR}(i, j)$ based a mathematic model in accordance with embodiments of the present inventive concept to reduce the static background in the speckle contrast as illustrated in Figures 9A and 9B. Figures 9A and 9B illustrate perfusion measurement using only near infra-red light (9A) and dual wavelength illumination (9B) of a large bowel of a pig. Measurement inaccuracy caused by the static contrast can be seen on the surgical drape 950 in Figure 9A. In Figure 9B, the "fake" blood flow and perfusion is not visible on the surgical drape 950 due to reduction of the static contrast.

Embodiments of the present inventive concept propose the visualization of both anatomical structure and blood flow physiology of the tissue and organ by one of two approaches. However, it will be understood that embodiments of the present inventive concept are not limited to the approaches discussed herein.

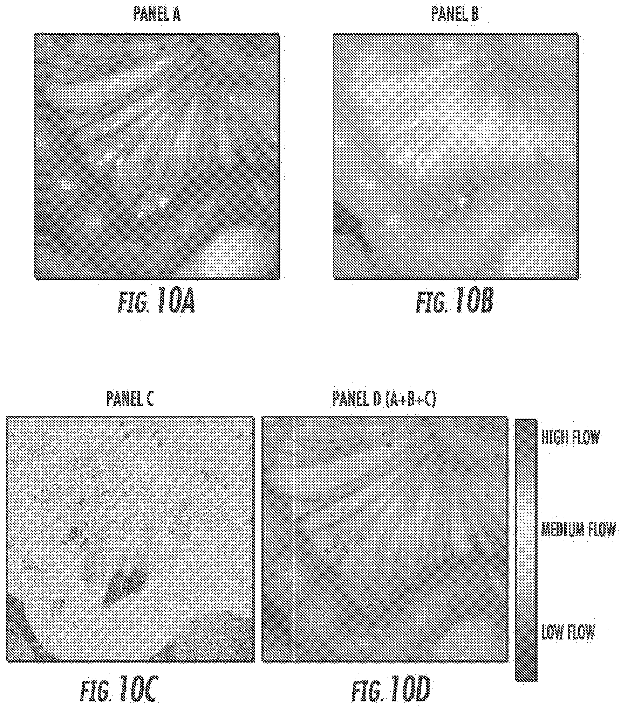

Referring now to Figure 10A-10D, a first approach using a dual layer design will be discussed. Referring first to Figure 10A (Panel A), an anatomical layer represented by a raw (original) image frame of visible light is illustrated.

(Anatomical layer) $Img_{VIS}(i, j)$ is an 8 bit gray scale visible image of the sample/target tissue/organ and i and j are the pixel indexes along the horizontal and vertical direction. In some embodiments, the brightness, contrast and gamma value of this image might be adjusted to achieve better visualization effect.

Referring now to Figure 10B, a processed image is produced based on one or more raw image frames of near infra-red light to reflect two-dimensional (2D) speed distribution of blood flow and perfusion of the imaged tissue/organ using Laser Speckle or Laser Doppler Imaging technology. (Physiological layer) $Img_{NIR}(i, j)$ is an 8 bit indexed image with its numerical values mapped to a predefined color map. Usually, the color ranges from blue to red (0 to 255) with blue representing no/minimum flow speed and red representing the highest flow speed that the system can detect.

Referring now to Figure 10C, a transparency map is produced using methods that overlap the anatomical layer or parts of the anatomical layer over a physiological one, which will cause the bottom layer to be invisible (covered) or partially invisible (covered). Methods that overlap the physiological layer or parts of the physiological layer over anatomical one will cause the bottom layer to be invisible (covered) or partially invisible (covered). A transparency map/matrix is applied in accordance with embodiments of the present inventive concept to ensure the visibility of both layers using the following equation:

$$T(i, j) = \left(\frac{Img(i,j) - Min(Img(i,j))}{Max(Img(i,j)) - Min(Img(i,j))}\right)^x \qquad \text{Eqn. (4)}$$

where $T(i, j)$ is the transparency map with $Img$ being a raw (original) image frame of visible or near infra-red light and x being an adjustable parameter >0 and <=2. Basically, each pixel value in T(i,j) is between 0 and 1 with 0 representing no transparency and 1 representing 100% transparency. Parameter x controls the contrast of the transparency map and if x > 1, transparency has a larger dynamic range and if x < 1, the transparency has a smaller dynamic range. Figure 10D represents the combined visual effect using A, B and C in accordance with embodiments of the present inventive concept to reveal both anatomical structure and physiology.

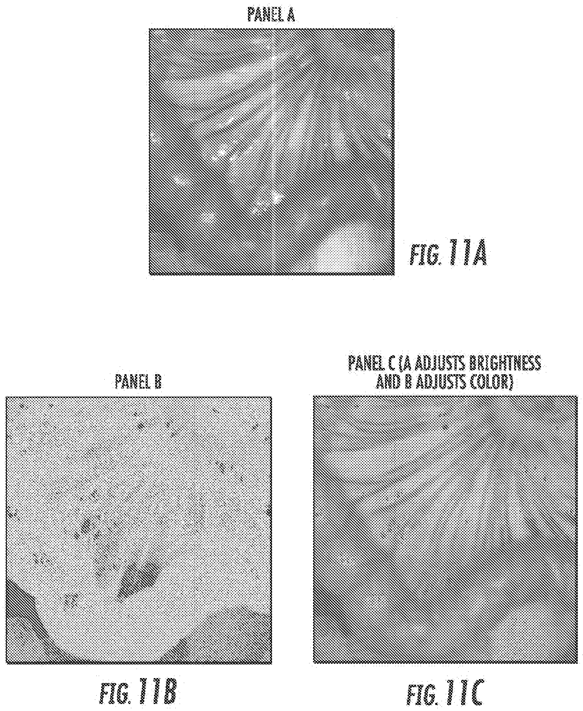

Referring now to Figures 11A through 11C, a second approach using color and brightness design will be discussed. As illustrated in Figure 11A, an anatomical layer is represented by image brightness: a raw (original) image frame of visible light. $Img_{VIS}(i, j)$ is an 8 bit gray scale visible image of the sample/target tissue/organ and i and j are the pixel indexes along horizontal and vertical direction. The brightness, contrast and gamma value of this image may be adjusted to achieve better visualization effect.

Referring now to Figure 11B, a physiological layer is represented by image color: a processed image based on one or more raw image frames of near infra-red light to reflect 2D speed distribution of blood flow velocity and perfusion of the imaged tissue/organ using Laser Speckle or Laser Doppler Imaging technology. In a first step, an 8 bit indexed color image is generated with its numerical values mapped to a predefined color map. Usually, the color ranges from blue to red (0 to 255) with blue representing no/minimum flow speed and red representing the highest flow speed that the system can detect. In a second step, the 8 bit indexed color image is converted to a normalized RGB map $RGB_{NIR}(i,j)$ with the color of each pixel being represented by (R, G, B) three values and each value range from 0 ~ 1. It will be understood that since the Figures are in black and white, the corresponding grey scale has been employed herein.

Referring now to Figure 11C, anatomical and physiological layers are fused together by creating an 8 bit RGB color image as $Img(i, j) = Img_{VIS}(i, j) \times RGB_{NIR}(i, j)$. Note, each color channel (matrix $R_{NIR}(i, j)$, , $G_{NIR}(i, j)$ and $B_{NIR}(i, j)$) is multiplied by the same visible image $Img_{VIS}(i, j)$.

According to some embodiments of the present inventive concept, multi wavelength imaging design may be used to simultaneously combine different imaging technologies together. For example, as discussed herein, NIR fluorescence technology based on indocyanine green uses 808nm illumination and the fluorescence emission light is 830nm and 808nm reflection light is considered as noise and filtered out. In accordance with some embodiments of the present inventive concept, the 808nm reflection light can be used to achieve LSI or LDI while maintaining the 830nm fluorescence function.

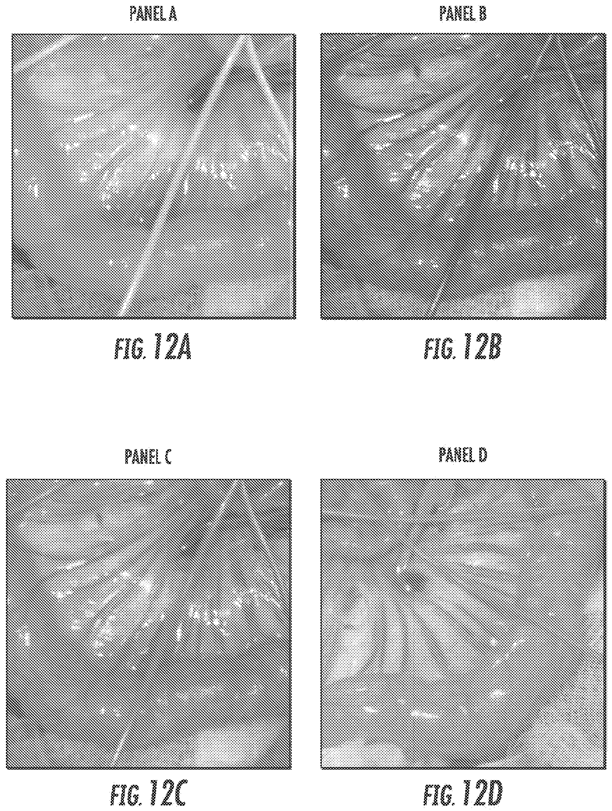

Referring now to Figures 12A-12D, images illustrating Panel A, an NIR 785nm image of a small bowel (12A); Panel B a Green 532nm image of the same small bowel (12B); Panel C, a reconstructed color image of the same small bowel (12C); and Panel D, an image of the same small bowel taken by a regular camera (12D) will be discussed. In particular, using the multi spectral imaging system in accordance with some embodiments of the present inventive concept, an original color image can be constructed by using each spectrum as one RGB color channel. For example, using an NIR image as a red color channel and a 532nm image as a green color channel, the color image of a small intestine can be generated without using a color camera as illustrated in Figures 12A-12D. It will be understood that since the Figures are black and white, the corresponding grey scale has been employed herein.

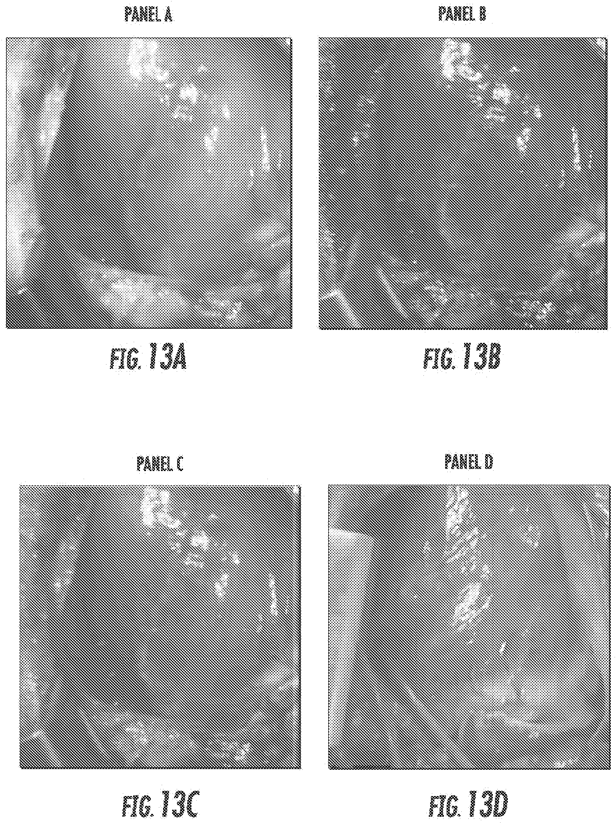

Referring now to Figures 13A-13D, images illustrating Panel A, an NIR 785nm image of a pig heart (13A); Panel B, Green 532nm image of the same pig heart (13B); Panel C, a reconstructed color image of the same pig heart (13C); and Panel D, an image of the same pig heart taken by a regular camera (13D) will be discussed. Figures 13A-13D illustrate using an NIR image as a red color channel and a 532nm image as a green color channel, the color image of a pig heart can be generated without using a color camera. If the information of one color channel is missing, an algorithm is designed to generate this data using the information of the other two color channels. Since the color of a sample (tissue/organ) is mainly red, embodiments of the present inventive concept can generate color that is very close to the original one as long as the information of the red color channel is available as discussed with respect to Figures 10A-10D and 11A-11D. Thus, embodiments of the present inventive concept allow the reconstructed color image to reveal information of deeper tissue/organ if NIR is used as the red color channel as shown in Panel C (Figure 12C) vs. Panel D (Figure 12D).

As discussed briefly above with respect to the Figures, some embodiments of the present inventive concept use two wavelengths of differential transmittance through target tissue to apply LSI or LDI. In some embodiments, a first wavelength is within the visible range having zero or very shallow penetration, such as blue light (450-495 nm). The imaging result of this non-penetrating illumination serves as capturing the anatomical structure of tissue/organ surface and position marker of the target tissue/organ, but not the subsurface movement of blood flow and perfusion. A second of the two wavelengths is Near Infra-Red (NIR), which has much deeper penetration and the imaging result of this NIR illumination reveals the underlying blood flow physiology, which correlates both to the motion of the target tissue/organ and also the movement of blood flow and perfusion.

Using the imaging measurement of the visible light as a baseline, the true motion of blood flow and perfusion can be derived from the NIR imaging measurement without being affected by the motion artifact of the target. Furthermore, the anatomical structure information captured by visible light and the physiological characteristics measured by NIR light may be synthesized together according to some embodiments of the present inventive concept. The synthesized imaging product according to embodiments discussed herein provides a previously unattainable clarity of visualization and accuracy of quantification of blood flow and perfusion across the spectrum of clinical applications of laser imaging technologies.

Thus, embodiments of the present inventive concept provide improved image quality and real time data acquisition (several seconds vs. minutes for all other technologies) and analysis. This real time aspect of the present inventive concept makes this technology a real option for sustained adoption of the technology by a surgeon/provider. Embodiments of the present inventive concept accurately depict and quantify blood flow and perfusion.

Further embodiments of the present inventive concept are directed to color image reconstruction using multi-wavelength imaging techniques discussed herein. It will be understood that the images are presented in a gray scale as the patent application publishes in black and white. In particular, using a dual wavelength imaging technique as discussed herein, two images may be acquired simultaneously. One is near infra-red image $IR(x, y)$ and the other is a visible image $VIS(x, y)$. X and Y represent the index of the horizontal and vertical pixel. To reconstruct a red green blue (RGB) color image, red, green and blue channels are calculated separately as follows:

$$R(x,y) = (2^N - 1) \times a_1 \times \left(\frac{NIR(x,y) - \min\left(NIR(x,y)\right)}{\max\left(NIR(x,y)\right) - \min\left(NIR(x,y)\right)}\right)^{b_1} \quad \text{Eqn. (5)}$$

$$G(x,y) = (2^N - 1) \times a_2 \times \left(\frac{VIS(x,y) - \min\left(VIS(x,y)\right)}{\max\left(VIS(x,y)\right) - \min\left(VIS(x,y)\right)}\right)^{b_2} \quad \text{Eqn. (6)}$$

$$B(x,y) = (2^N - 1) \times a_3 \times \left(\frac{VIS(x,y) - \min\left(VIS(x,y)\right)}{\max\left(VIS(x,y)\right) - \min\left(VIS(x,y)\right)}\right)^{b_3} \quad \text{Eqn. (7)}$$

$$\frac{NIR(x,y) - \min\left(NIR(x,y)\right)}{\max\left(NIR(x,y)\right) - \min\left(NIR(x,y)\right)} \qquad \text{Eqn. (8)}$$

where R(x,y), G(x,y), B(x,y) are the red, green and blue channels, respectively, of the RGB color image; N is the bit of the color map, for example, 8 bit or 16 bit; a and b are the adjusting parameters for each channel; min is the function to get the minimum value; max is the function to get the maximum value; and Eqn. (8) serves as a normalization of the original image of one specific wavelength. Furthermore, the brightness, contrast and gamma value of the original image of one specific wavelength might be adjusted before applying the equations above.

The multi-wavelength color image recreation technique in accordance with some embodiments of the present inventive concept may reduce the need for an extra color camera in the device; can create a color image with a minimum of two wavelengths; and compared with traditional color images, the color image produced in accordance with embodiments discussed herein visualizes a larger depth of penetration due to use of near infra-red wavelength.

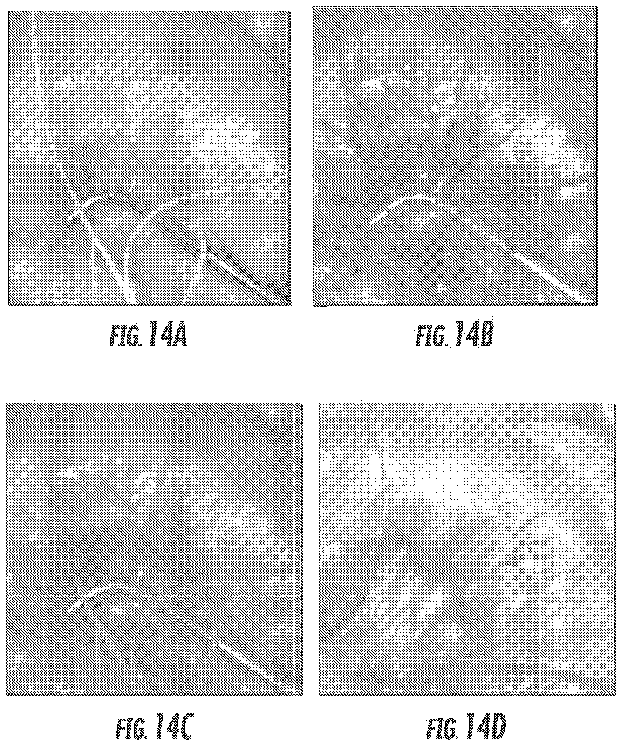

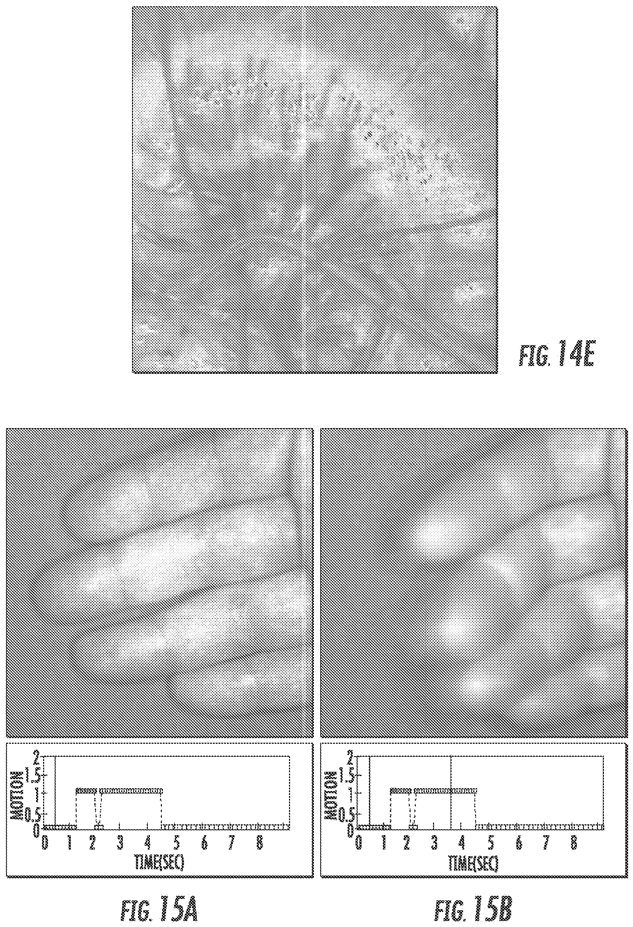

Referring now to Figures 14A through 14E, various images of a segment of a large bowel of a pig imaged using the multi-wavelength imaging device in accordance with some embodiments of the present inventive concept will be discussed. Figure 14A is an image of the bowel of the pig obtained using a visible wavelength (532nm). Figure 14B is an image of the bowel of the pig using a near infra-red wavelength (785nm). Figure 14C is an image of the bowel of the pig reconstructed with the wavelengths of Figures 14A and 14B. Figure 14D is a regular color image (shown in gray scale) of the bowel with room light illumination. Figure 14E is a blood flow and perfusion image of the bowel in accordance with some embodiments of the present inventive concept.

Referring now to Figures 15A to 19B, details with respect to real time image quality test protocols will be discussed. Real time image quality test protocols are developed based on customized algorithms using image registration and image metadata to examine the following issues during a clinical imaging procedure:

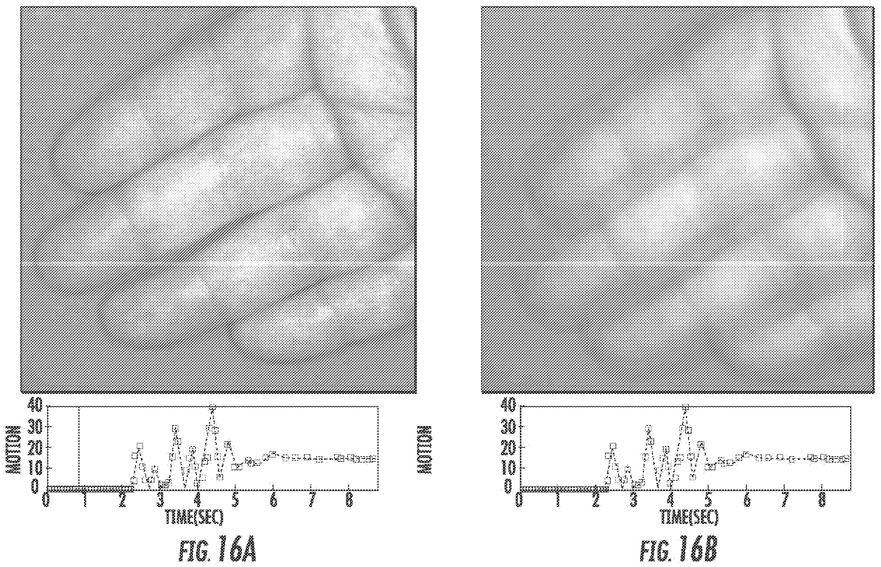

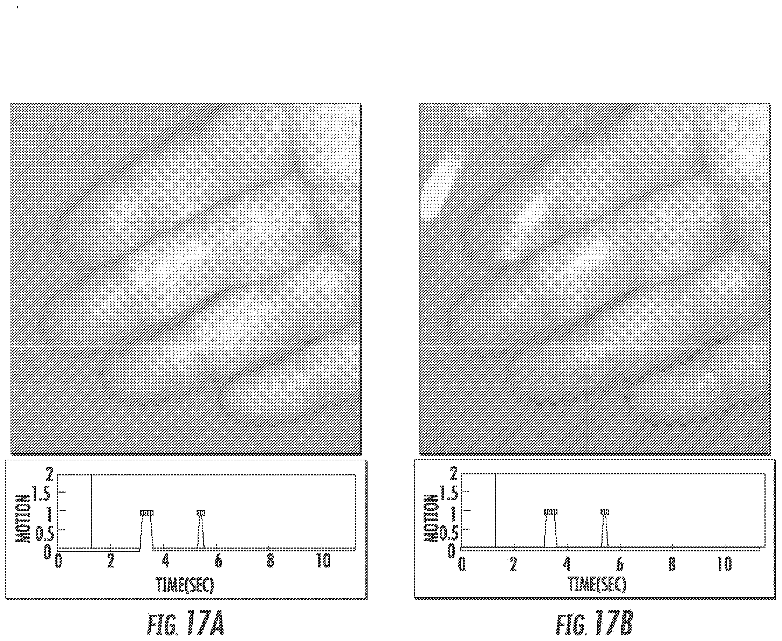

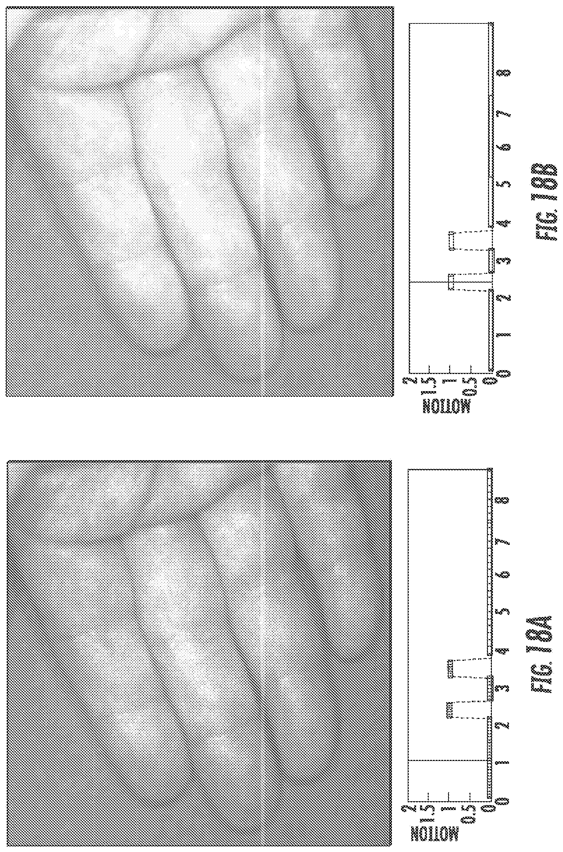

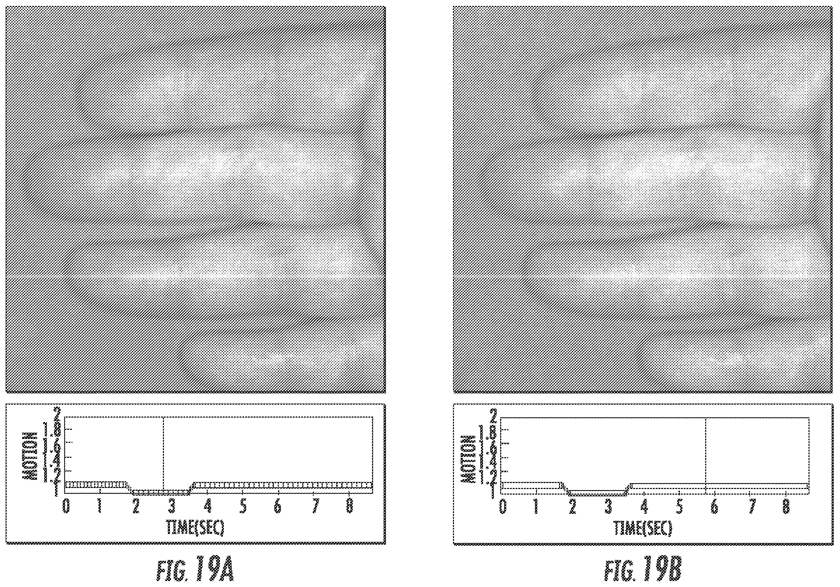

- Movement of target: Figures 15A and 15B illustrate images of a stationary hand (15A) and a moving hand (15B) detected by a customized image registration algorithm. By a customized image registration and optical flow algorithm, the quantified detection result curves can be drawn under the images.
- Movement of a field of view or the Camera: Figures 16A and 16B illustrate imaging of a hand image captured by stationary camera (16A) and a hand captured by moving camera (16B) detected by customized image registration algorithm.
- Blocked field of view: Figures 17A and 17B illustrate an image of a hand (17A) and an image of a hand that is partially blocked by a twister (17B) and this blocked field of view is detected by a customized image registration algorithm.
- Intrusion of headlight of surgeon/physician: Figures 18A and 18B illustrate an image of a hand (18A) and an image of a hand with a head light shining on it (18B) and this extra light within the FOV is detected by a customized algorithm using metadata in the image.
- Ambient light condition: Figures 19A and 19B illustrate an image of a hand with a room light off (19A) and an image of a hand image with the room light on (19B) and this is detected by customized algorithm using metadata in the image.

The goal of this process is to reduce the likelihood, or possibly eliminate, low quality images caused by incorrect image acquisition to improve the visualization and increase accuracy of the quantification of the blood flow and perfusion imaging in accordance with some embodiments of the present inventive concept.

As discussed above, the data obtained using the imaging methods discussed above can only be used to derive distribution of blood flow speed u. In clinics, the information on distribution of blood flow rate given by the product of blood flow velocity u and the cross section area of blood vessel A is needed. To obtain the distribution of u(r) where r is the three dimensional coordinate, the Navier-Stokes equation has to be solved, which is given by Equations (9) and (10) set out below:

$$\rho \cdot (\frac{\partial \mathbf{u}}{\partial t} + \mathbf{u}\nabla \cdot \mathbf{u}) = -\nabla p + \mu \cdot \nabla^2 \mathbf{u} + \mathbf{F} \qquad \text{Eqn. (9)}$$

$$\frac{\partial \rho}{\partial t} + \nabla \cdot (\rho \mathbf{u}) = 0 \qquad \text{Eqn. (10)}$$

where $\rho$ is the density (kg/m$^3$), u is the flow velocity vector (m/s), p is the pressure (N/m$^2$ or Pascal), F is the volume force vector (N/m$^3$) and $\mu$ is the viscosity. Solving the Navier-Stokes equations produces a velocity field, *i.e.* a distribution of fluid velocity in space and time. Once this velocity field is obtained, other quantities of interest, such as flow rate and drag force, can be calculated. These calculated quantities can be compared to the experimental data obtained using the methods discussed above to validate the data.

Computational procedures for a non-invasive measurement of blood flow rate distribution in principal vessels in tissues/organs will now be discussed with respect to some embodiments of the present inventive concept. Procedures begin by illuminating a tissue region of interest with a coherent light source, such as a laser with sufficiently long wavelength for relatively large penetration depth between, for example, 550 nm to about 1100 nm as the second wavelength. Using methods discussed above, scattered light at the second wavelength is acquired to determine spatial distribution of blood flow speed in the principal vessels and perfusion distribution in tissue in the region of interest. A velocity field of u(r) for the region of interest is calculated numerically. In some embodiments, the velocity field is calculated using Equations (9) and (10) set out above. Blood flow speed in the region of interest based on the calculated velocity field is calculated. The calculated blood flow speed in the region of interest is compared to the blood flow speed determined using the acquired image data at the second wavelength from the region of interest to verify results.

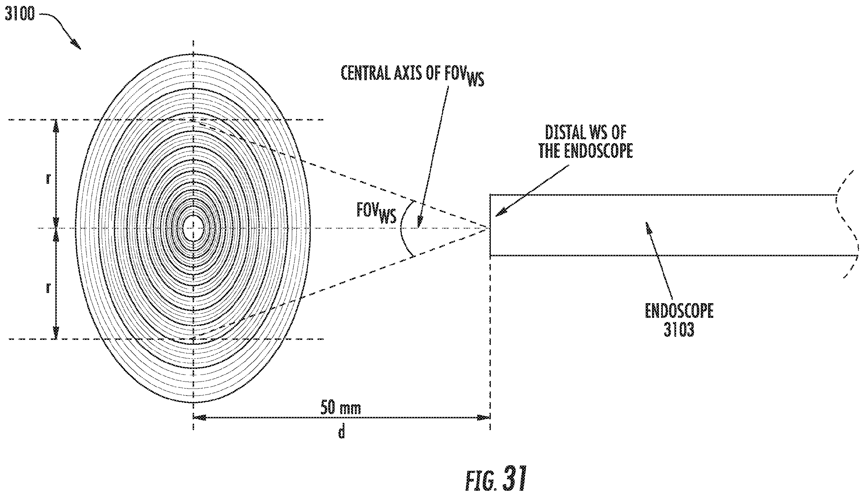

As used herein, "blood flow rate distribution" refers to a relation between velocity distribution of u (the velocity vector, in m/sec) in the region of interest or field of view (FOV) and the blood flow distribution. Calculation of blood flow rate distribution (volume flow in cc/min) involves using tools such as computational fluid dynamics models to obtain blood flow rate (a surface integral of the u vector over the cross section of a vessel) from a measured distribution of velocity u. Furthermore, embodiments of the present inventive concept are configured for macro FOVs of, for example, about 100 mm × about 100 mm. Details with respect to FOV for various embodiments of the present inventive concept are discussed further below with respect to Figure 31.

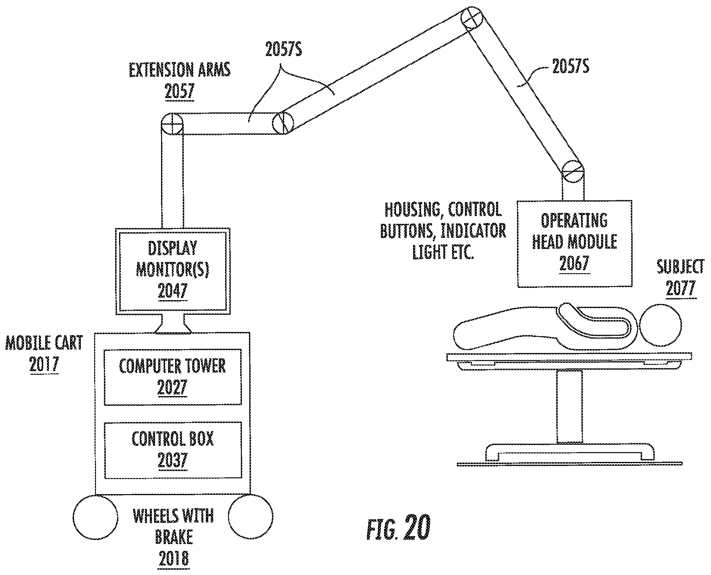

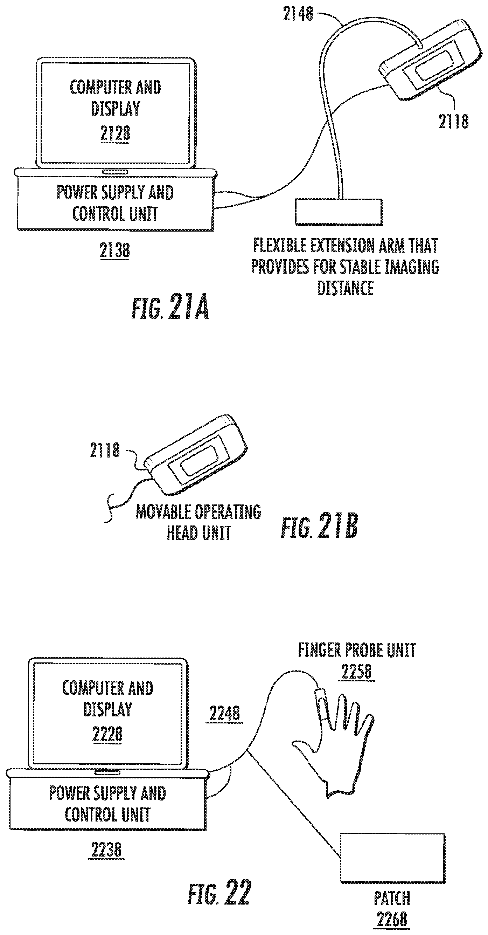

Referring now to Figures 20, 21A and 21B, various embodiments of non-invasive systems in accordance with embodiments of the present inventive concept will be discussed. Referring to Figure 20, a mobile system in accordance with embodiments of the present inventive concept will be discussed. As illustrated therein, the system includes a mobile cart 2017 including, for example, a communications device (computer tower) 2027, a control box 2037, a series of shelves in a housing etc., and a computer terminal or display 2047 thereon; an extension arm 2057 and an operating head module 2067 in accordance with embodiments discussed herein. The mobile cart 2017 may include wheels 2018 and a mechanism for locking the wheels, for example, a brake, when the mobile cart 2017 is positioned near the subject. The operating head module may include a housing, a user interface, lights and alarms and the like without departing from the scope of the present inventive concept. The extension arm 2057 may be used to position the operating head module 2067 over the patient/subject 2077. The ability to move the system to the patient, rather than bring the patient to the system provides many advantages, especially for immobile patients. In some embodiments, the extension arm 2057 can including a plurality of articulating arm segments 2057s.

Referring now to Figures 21A and 21B, smaller footprint embodiments in accordance with inventive concept will be discussed. As illustrated in Figures 21A and 21B, a smaller footprint operating head unit may be used to provide operations in accordance with embodiments discussed herein. These systems may include a communications device 2128 including a computer console and related display and user interface, a power supply and a control unit 2138 all coupled to a handheld operating head unit 2118. As illustrated in Figure 21A, in some embodiments the smaller footprint mobile operating head unit 2118 is coupled to the communications device 2128 using a flexible extension arm 2148. The flexible extension arm 2148 may allow the mobile operating head unit 2118 to be positioned on the target region of the patient/subject without repositioning the patient subject. Figure 21B illustrates a mobile operating head unit 2118 without a flexible extension arm 2148. It will be understood that Figures 21A and 21B are provided as examples only and, therefore, embodiments of the present inventive concept are not limited to this configuration.

Referring now to Figure 22, finger probe/skin patch embodiments of the inventive concept will now be discussed. As illustrated in Figure 22, these systems may include a communications device 2228 including a computer console and related display and user interface, a power supply and a control unit 2238 all coupled to a finger probe unit 2258 and/or a skin patch 2268. As illustrated in Figure 22, in some embodiments the finger probe unit 2258 or the skin patch 2268 is coupled to the communications device 2228 using a flexible extension arm and/or cable 2248. The flexible extension arm 2248 allows the finger probe unit 2258 and/or the skin patch 2268 to be positioned on a finger or skin of the subject without repositioning the subject. It will be understood that Figure 22 is provided as an example only and, therefore, embodiments of the present inventive concept are not limited to this configuration.

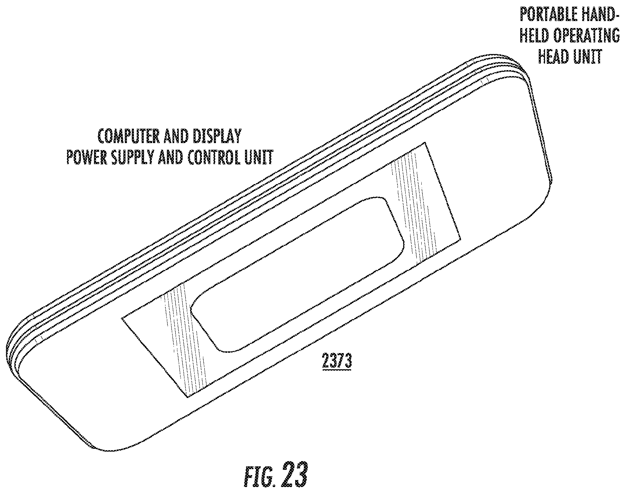

Referring now to Figure 23, hand-held mobile embodiments of the present inventive concept will now be discussed. As illustrated in Figure 23, these systems may include a communications device 2373 including, for example, a microchip computer and related display and user interface (as shown in Figure 21A), a battery power supply and a control unit. The image is captured non-invasively at a focused distance or by contact with a surface of the handheld unit. Embodiments may include image capture, analysis, display and storage as discussed above. Wireless communication protocols may be used to download the stored information without departing from the scope of the present inventive concept.

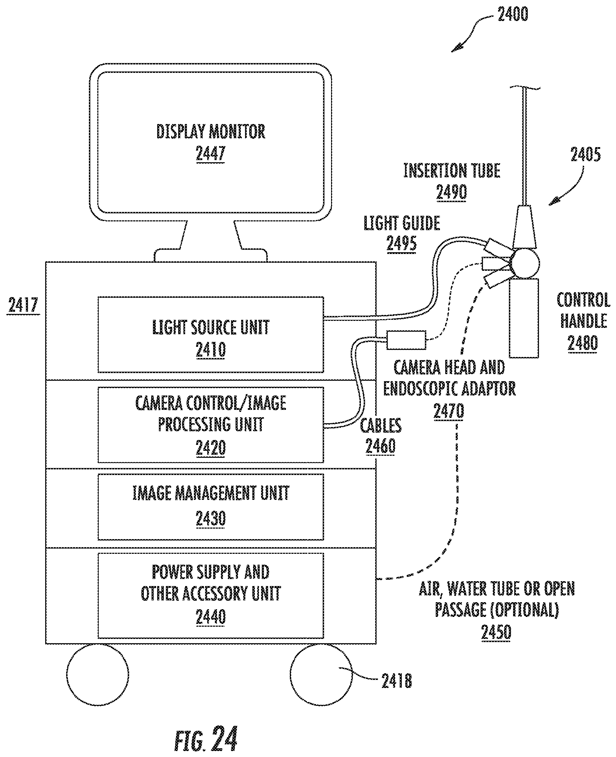

As is clear from details discussed herein and Figures 20 through 23, there are common components that are included in the various embodiments and form factors discussed herein. In particular, the system generally includes optics including a camera that can be a customized multi-sensor device; multiple near infrared and visible lasers; a light emitting device (LED)(for example, 2410, Figure 24) to locate a field of view (FOV); distance sensors to mark the distance to ensure the appropriate size of the field of view; and fiber and other front end optics.

Most embodiments include a communications device, *i.e.* a computer and display unit to process the raw image from the camera and visualize the blood flow and perfusion in tissue/organ and communicate with the electronic control board.

The systems are coupled to a power supply and a control unit or any combination thereof. For example, an alternating current (AC), direct current (DC) or battery power supply provides power for different components in the system. Furthermore, an electronic control board can control different components and read feedback and communicate with the computer through software.

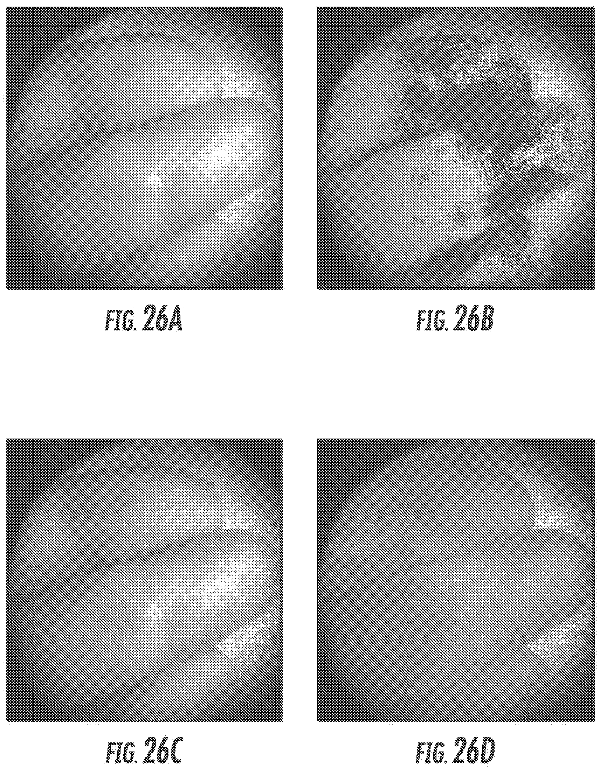

Some embodiments of the present inventive concept provide real time software analysis and visualization to provide simultaneous anatomical and blood flow and perfusion physiological views as illustrated, for example, in Figures 26A through 26D. It will be understood that for each embodiment (form factor) the key components and output may vary without departing from the scope of the present inventive concept. For example, in the finger probe application (Figure 22), the numbers and measurement curve will be used instead of image data.

Referring now to Figures 26A through 26D, images obtained using methods and systems in accordance with embodiments discussed herein will be discussed. Figures 26A and 26B are an anatomical view and a blood flow physiology of a normal human hand, respectively. Figures 26C and 26D are an anatomical view and a blood flow physiology of an abnormal human hand with insufficient blood supply, respectively.

As discussed above, some embodiments of the present inventive concept provide methods, systems and computer program products for non-invasive determination of blood flow and perfusion in a sample. Although multi-spectral physiologic visualization (MSPV) can be used in a non-invasive, open surgical setting with direct illumination of the tissues, embodiments of the present inventive concept are not limited to this configuration. In particular, although not covered by the claimed method, recently many surgical procedures have been performed using endoscopy-based illumination for visual access within cavities of the body. According to some embodiments of the present inventive concept, MSPV can be adapted for an endoscopic platform, which includes, for example, endoscopic cavitary surgery, minimally invasive surgery, and robotic surgery, all of which use endoscopic visualization to illuminate the tissues and organs and to perform the procedure as will be discussed further herein.

As used herein, an "endoscopic system" refers to both an endoscope-type device or placement of an endoscope-type device into a cavity, recess, natural lumen or tube-like structure in the subject, for example, the human body.

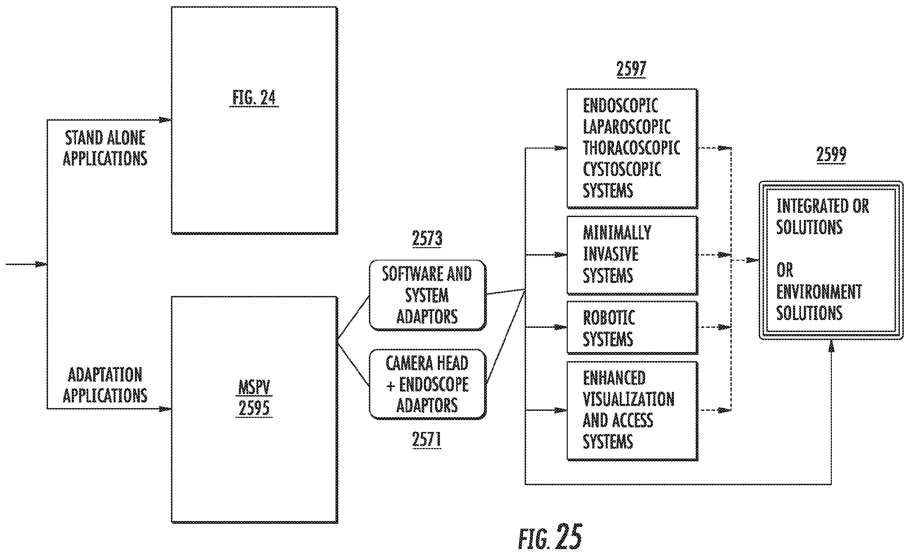

Referring now to Figure 24, a standalone MSVP endoscopic system 2400 including an endoscopic device 2405 in accordance with some embodiments of the present inventive concept will be discussed. As will be discussed further below with respect to Figure 25, embodiments of the present inventive concept are not limited to standalone systems. Referring to Figure 24, the system includes a mobile cart 2417 including, for example, a light source 2410; a camera control and image processing unit 2420; an image management unit 2430; a power supply and other accessory unit 2440; a display 2447 and an endoscopy device 2405 coupled thereto in accordance with some embodiments discussed herein.

The endoscopy device 2405 includes a light guide 2495 coupled to the light source 2410, a camera head and endoscopic adaptor 2470 coupled to the camera control 2420 and an optional air and water tube 2450 coupled to the accessory unit 2440. The light guide 2495, the camera head and endoscopic adaptor 2470 and the air and water tube 2450 may be connected to a control handle 2480 of an insertion tube by cables 2460. The insertion tube may be flexible, semi-rigid or rigid as will be discussed below.

The mobile cart 2417 may include wheels 2418 and a mechanism for locking the wheels, for example, a brake, when the mobile cart 2417 is positioned near the subject. The mobile cart 2417 may also include shelving etc. for the various equipment modules included therein. Although embodiments of the present invention are illustrated in Figure 24 as being a mobile cart, it will be understood that embodiments of the present inventive concept are not limited to this configuration. For example, the equipment may be stationary or embedded within a larger integrated system (e.g., Operating Room suite systems) without departing from the scope of the present inventive concept. However, the ability to move the system to the patient, rather than bring the patient to the system provides many advantages, especially for immobile patients.

Details of the system illustrated in Figure 24 will now be discussed. As discussed above, some embodiments include a mobile cart 2417, which can be used to transport the whole endoscopic system into different units of the hospital or clinic area. Brakes can usually be applied when the system needs to be secured. Multiple shelves, brackets and holders may be designed to contain different modules, mount monitor and hold the endoscope and accessories.

The display monitor 2447 is not limited to the single monitor shown in Figure 24. For example, one or more monitors may be provided on the mobile cart 2417 without departing from the scope of the present inventive concept. The monitor (s) 2447 may connect to the camera control and image processing unit 2420 to be viewed by, for example, physicians or nurses, in real time. The monitor(s)2447 may also connect to the image management unit 2430 to be viewed for case and episode management. In further embodiments, the monitor 2447 may be provided on the display on the endoscope itself. In some embodiments, an adaptor mat be provided in the system that allows the system to be connected to existing monitors in the operating room (OR).

The light source 2410 may include a multi-spectral LED light to guide the field of view (FOV) of the endoscope insertion process. The light source 2410 may also include one or more multi-spectral lasers for blood flow and perfusion visualization once the region of interest (ROI) is located. The light source unit 2410 is generally connected to the endoscope with optic fibers. Embodiments of the present inventive concept may provide a light source 2410 specifically configured to work in endoscopic environments.

The camera control and image processing unit 2420 receives image data from the camera sensors that are located in the endoscope. An embedded processing system (different from PC) will process the raw data using algorithms and the multi-spectral perfusion visualization (MPSV) and regular color visualization will be sent to the display monitor 2447. The embedded processing system may also control the camera sensors in the endoscope and adjust the camera settings. In some embodiments, the camera head 2470 (or part of the camera head) can be located in the camera control and image processing unit to reduce the profile of the endoscope. In some embodiments, the camera is specifically designed for endoscopic environments accordance with those discussed herein.

The image management unit 2430 may be configured to provide a user interface for case and episode management; image file management, for example, data archiving and exporting; and other system management such as network connectivity. The image management unit 2430 may be achieved by a PC-style architecture with, for example, a Windows or LINUX operating system. It will be understood that the image management unit can be incorporated into the camera control and image processing unit 2420 to make the endoscopic system more compact without departing from the scope of the present inventive concept. However, the two units may also remain separate.

The power supply 2440 is physically isolated from the electrical plug for safety reasons. The other accessories such as water, air supply and suction pump etc. may be needed depending on the specific clinical application.

The endoscope 2405 is provided by the insertion tube 2490, the light guide 2495, the camera head and endoscopic adaptor 2470, the cables 2460, the optional air, water, or open passage tubes 2450 (dotted lines indicate the optional elements) and the control and handle 2480. It will be understood that in some embodiments, flexible scopes may have air, water, and open passage (e.g., a biopsy channel) and may not have one or more of these elements in another embodiments. Rigid scopes may have an open channel, but may not have not air /water. For laparoscopic/thoracoscopic/robotic endoscopes, there are typically no channels in the rigid scopes.

Details of these elements of the endoscopic system 2400 will now be discussed. Some embodiments provide two type of insertion tubes 2490, rigid and flexible. It will be understood that in some embodiments the insertion tube could be semi-rigid without departing from the scope of the present inventive concept. Each contains an optical lens and/or fiber bundles to illuminate the target with LED and near infrared laser light and receive light that is reflected from the target. The insertion tube may also include water, air and suction channels depending on the specific clinical application.

A flexible insertion tube may, for example, include a light guide connector, a light guide tube, video remote switches, a control body, a bending section of the insertion tube which may include internal instrument channels. A rigid endoscope may include, for example, an eyepiece, an eyepiece sleeve, fiber optics, a fiber guide, a light cone and sleeve, a light post and rigid insertion tube with a distal tip. At an end of the rigid insertion tube an objective lens assembly may be provided.

Referring again to Figure 24, the light guide 2495 is plugged into the light source unit to transmit light. The camera head can be potentially located in, for example, an endoscopic adaptor 2470. The image may be carried from the distal tip of the endoscope to the camera head by lens and/or fiber bundles and then electronically transferred to the camera control and image processing unit 2420 through cable 2460.

The camera head (or part of the camera head) 2470 can be potentially located in the camera control and image processing unit 2420 to reduce the profile of the endoscope.

An image can be formed at the distal tip of the endoscope and/or image data can be obtained using image sensors (S1-SN - Figure 1C) and transmitted electronically to the camera control and image processing unit 2420. Different control buttons are located around the handle 2480 of the endoscope to achieve field of view and imaging control etc. during the operation procedure. For example, in some embodiments, the sensors S1-SN (Figure 1C) may be arranged along a continuum from the tip of the endoscope to a distance remote from the endoscope coupled by image capture cabling.

Referring now to Figure 25, as discussed above, embodiments of the present inventive concept are not limited to a standalone system discussed with respect to Figure 24. Figure 25 is a block diagram illustrating both a standalone system and adapted system. The standalone system illustrated in Figure 25 is similar to the standalone systems illustrated and discussed with respect to Figure 24. Accordingly, the details will not be repeated herein in the interest of brevity.

When the adaptation applications are used, the MSVP system 2595 interfaces with a camera head and endoscopic adapters 2571. Software applications 2573 are provided interface with system adapters and system software. Both the hardware 2571 and the software 2573 of the adaptive system can be connected to any system 2579, for example, endoscopic systems, thoracoscopic systems, minimally invasive systems, robotic systems, enhanced visualization and access systems, and the like. Thus, existing systems can use the inventive concept discussed herein. In further embodiments, the existing systems can be interfaced into Integrated OR systems (OR Environment Solutions) 2599. It will be understood that embodiments of the present inventive concept are not limited to embodiments illustrated in Figure 25, which is provided for example only. For example, blocks illustrated in Figure 25 may be combined or expanded without departing from the scope of the present inventive concept. For example, although block 2753 is illustrated as having three functions, these functions can be separated into two or more blocks.

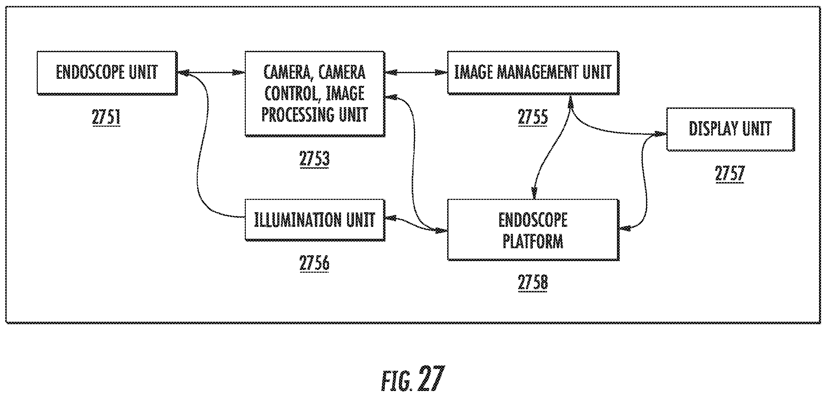

A block diagram of an endoscopic system in accordance with some embodiments of the present inventive concept is illustrated in Figure 27. As shown, the endoscopic system includes an endoscopic unit 2751; a camera 2753; a camera control 2753; an image processing unit 2753; an illumination unit 2756; an image management unit 2755; an endoscopic platform 2758 and a display unit 2757. Figure 27 is provided for example only and, thus, embodiments of the present inventive concept are not limited to this configuration. Units or modules may be combined or expanded into multiple units without departing from the scope of the present inventive concept.

In some embodiments, one or more cameras may be used. A single camera may have one or more sensors available.

In the adaptation of MPSV for an endoscopic application, several modifications are considered. The adapted endoscopic system components may include a modified endoscope technology, a camera, camera control and image processing unit, an image management unit, an operating system interface with the endoscopic platform operating system, and an interface with the endoscopic platform display(s).

In some embodiments, the illumination source is modified to accommodate the illumination path to include an endoscope

In some embodiments, the illumination path is modified to produce a highly coherent beam. In further embodiments, this beam is diffused equally over the FOV, is focused at a certain distance, and provides high image quality and fidelity.

In still further embodiments the illumination source is multiple lasers, but can also include non-laser high-energy LEDs. Both laser and LED illumination may be used.

In some embodiments, the illumination light path travels from the source out to the target through the endoscope fiber array that consists of multiple wavelength-specific illumination fibers as well as image capture fibers.

In some embodiments, the camera is a miniaturized multi-sensor camera affixed to the endoscope. In other embodiments, the camera system is connected via cable to the endoscope. In these embodiments, the reflected and scattered light is captured by the camera with minimal loss through the fiber connectivity between the endoscope tip and the camera. In some of these embodiments, the camera will be a variable distance from the endoscope body to facilitate incorporation into existing endoscope-based platforms.

In some embodiments, the camera speed is about 150 frames per second to effect imaging fidelity and real-time analyses.

In some embodiments, the MSPV operational software (but not the MSPV image capture or the MSPV analysis software) will be integrated into the endoscope-based platform operational software in order to work seamlessly together. This interface with the endoscope-based platforms includes the platforms of endocavitary surgery such as laparoscopic surgery, thoracoscoic surgery and others inside a body cavity, minimally invasive surgery platforms such as minimally invasive Coronary Artery Bypass Graft surgery, and endoscope-based robotic surgical platforms.

In other embodiments, the image display quality is high definition (HD), three-dimensional (3-D), or four-dimensional (4D) and organic light emitting diode (OLED), all compatible with the MSPV analyses presentation.

In some embodiments the endoscope design is a rigid scope, with a straight or an angled lens at the tip.

In some embodiments the camera chip can be localized at the endoscope tip.

In some embodiments the endoscope design is a rigid scope with a flexible tip, where the camera chip and lenses are in the distal 2.0 cm of the 'rigid' scope; this distal 1.0 cm is flexible with a 360 degree radial field of view.

In some embodiments the scope is a flexible endoscope for endoscopic, bronchoscopic and similar 'scopic' procedures, incorporating this scope design modified technically to enable MSPV imaging and analysis in real-time.

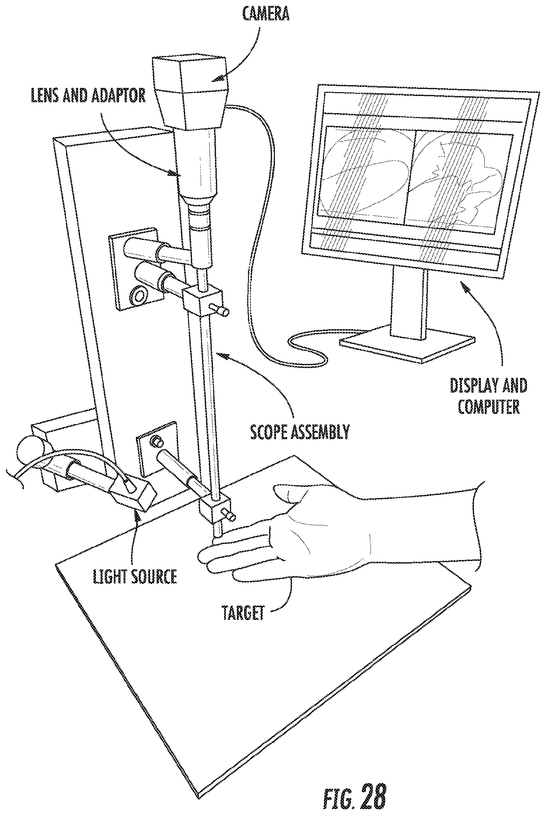

Figure 28 is an example of an actual system in a lab including some or all of the components discussed above with respect to Figure 27. Experiments have been performed using this system. The experiment setup of simultaneous reflectance imaging (illustrating anatomical structure) and near infrared laser speckle imaging (illustrating blood flow and perfusion distribution map) through a rigid laparoscope on optics bench top is illustrated in Figure 28. This experiment can be accomplished with one or multiple wavelength illumination and imaging in real time. For example, in some embodiments, the light source shines one or multiple wavelength light with a homogenous beam profile on a target (fingers) and the diffused reflected light is imaged through a rigid endoscope, lens and adaptor and imaged on a single sensor or multiple-sensor camera(s). The raw image data is transferred to the computer and processed using Eqns. 1 and 3 (for laser speckle imaging) and Eqns. 2 and 3 (for laser Doppler imaging). The anatomical structure and blood flow and perfusion distribution map are generated by algorithms discussed above with respect to Figures 10A-10D and Eqn. 4 and Figures 11A-11C and displayed on the monitor in real time as shown in Figure 2. Note that in this experiment the illumination light is delivered outside the endoscope and in practice the illumination light (one wavelength or multiple wavelength) can be delivered through the endoscope (rigid or flexible) using fiber bundle, micro lens and diffuser. This experiment is provided for example only and is not intended to limit the scope of the inventive concept.

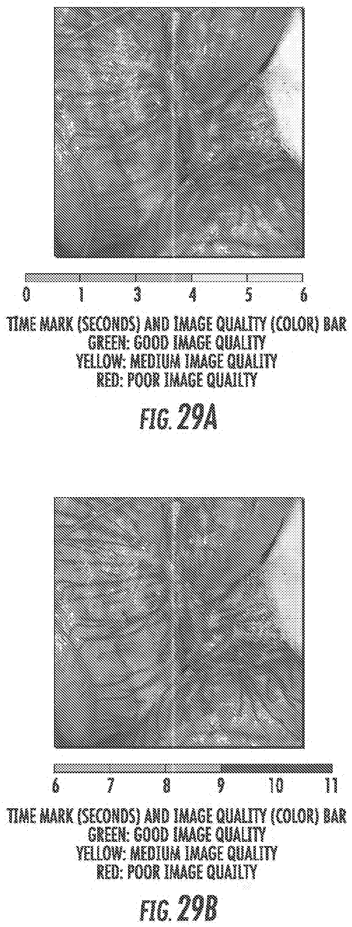

Referring now to the images and quality scale illustrated in Figures 29A and 29B. When MSPV videos, anatomical structure (Figure 29A) and blood flow and perfusion distribution map (Figure 29B) are displayed in real time or played back, a red(9-11); yellow (4-6); and green (0-4 and 6-9) colored time line bar (bottom) shown in black and white herein with numbers 0-11 can be used to indicate the time mark and the image quality at that time mark. The image quality checking results can be accomplished and embedded in the MSPV viewing section in real time or retrospectively in review mode without departing from the scope of the present inventive concept. In some embodiments, the image quality indicator bar is combined with the time line bar while the user is viewing the image in real time or retrospectively. The colors (red, yellow, green) or numbers 1-11 indicate the quality of video at that specific time mark. For example, green (0-4 and 6-9) may indicate a good quality image; yellow (4-6) may indicate a medium quality image and red (9-11) may indicate a poor quality image. Embodiments are not limited to the specific colors, numbers or scale illustrated in Figures 29A and 29B. Any scale can be used without departing from the scope of the present inventive concept.

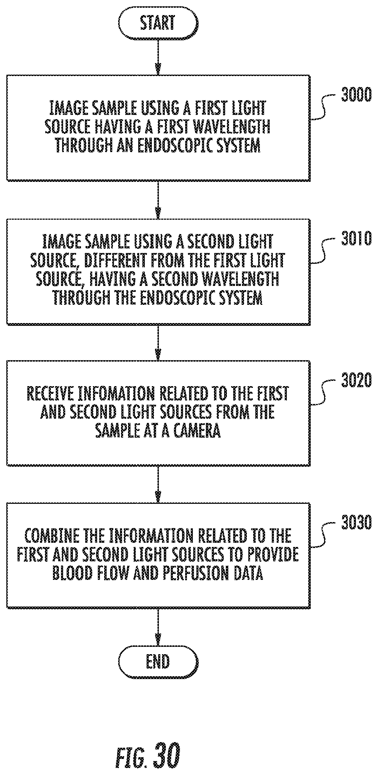

Referring now to Figure 30, a flowchart illustrating methods in accordance with some embodiments of the present inventive concept will be discussed. Operations for multispectral imaging in a multispectral imaging system adapted for an endoscopic system begin at block 3000 by imaging a sample using a first light source having a first wavelength and being delivered through an endoscopic system. The sample is imaged using a second light source, different from the first light source, having a second wavelength, different from the first wavelength and being delivered through the endoscopic system (block 3010). Receive information related to the first and second light sources from the sample at a camera (block 3020). The light at the first wavelength is configured to reflect off a surface of the sample into the camera and light at the second wavelength is configured to penetrate the sample and provide information related to the sample to the camera through the endoscopic system. The information related to the first and second light sources provided by the camera are combined at a processor to image an anatomical structure of the sample, image physiology of blood flow and perfusion of the sample and/or synthesize the anatomical structure and the physiology of blood flow and perfusion of the sample in terms of a blood flow rate distribution (block 3030). The illumination, analysis and display may be performed using the endoscopic system. The endoscopic system may be configured to communicate with a multi-spectral physiologic visualization (MSPV) operating system.

Referring now to Figure 31, a diagram illustrating details with respect to FOV in various embodiments of the present inventive concept will be discussed. Figure 31 illustrates a viewing circle 3100 associated with an endoscope 3103. The angular FOV ($FOV_{WS}$) is depicted in degrees. The radius r of the viewing circle 3100 is directly related to linear FOV as discussed below with respect to Eqn. 11. As illustrated in Figure 31, the object distance d is 50 mm in the example illustrated therein.

Generally, the FOV may be specified in one of the two ways: angular FOV specified in degrees; and linear FOV ($FOV_{WS}$) specified in the lengths/ratio of the lengths. With object distance and/or working distance being defined, the angular/linear FOV can be converted to the other using, for example, Eqn. 11 discussed below. The FOV of an endoscope 3103 is measured by the cone angle $FOV_{ws}$ as shown in Figure 21, which is the angular FOV. A cone angle $FOV_{WS}$ of a rigid endoscope can range from about 0 to about 90 degrees, typically about 30 degrees.

Example embodiments of the present inventive concept have used an Olympus 10mm rigid laparoscope having a 75 degree FOV. By changing the working distance, the linear FOV can range from under 1.0cm×1.0 cm to above 10.0cm×10.0 cm according to Eqn. 11.

$$FOV_{WS} = 2\text{atan}\left(\frac{r}{d}\right) \qquad \text{Eqn. (11);}$$

where $FOV_{WS}$ is the angular FOV in degrees; r is the radius of the viewing circle, which is directly related to linear FOV; and d is the object distance.

By using an appropriate optical adaptor lens, embodiments of the present inventive concept can be used to design a system having from 0 to 180 degree angular FOV and the corresponding linear FOV can be derived using Eqn. 11 set out above.

In the drawings and specification, there have been disclosed example embodiments of the inventive concept. Although specific terms are employed, they are used in a generic and descriptive sense only and not for purposes of limitation, the scope of the inventive concept being defined by the following claims.

**Claims**

1. A multispectral imaging system, the system comprising:

   a first light (130) source having a first wavelength configured to image a sample (160);
   a second light source (131), different from the first light source, having a second wavelength, different from the first wavelength, configured to image the sample (160);
   at least a third light source (132), different from the first and second light sources, having a third wavelength, different from the first and second wavelengths, configured to image the sample (160);
   a multi-sensor camera (110) having a plurality of sensors, each of the plurality of sensors configured to receive information related to one of the first, second and at least third light sources at a same time from the sample (160), wherein light at the first wavelength is configured to image a surface of the sample (160) into a first of the plurality of sensors of the multi-sensor camera (110); wherein light at the second wavelength is configured to penetrate the sample (160) to a first depth and provide information related to the sample (160) to a second of the plurality of sensors of the multi-sensor camera (110); and wherein light at the at least third wavelength is configured to penetrate the sample (160) at a second depth different from the first depth of the light at the second wavelength, and provide information related to the sample (160) to a third of the plurality of sensors of the multi-sensor camera (110), the information related to the first, second and third light sources being aligned and synchronized when provided to the first, second and third sensors of the multi-sensor camera (110); and

a processor configured to combine the information related to the first, second and at least third light sources provided by the multi-sensor camera (110) to (a) image an anatomical structure of the sample (160) and image physiology of blood flow and perfusion of the sample (160), and/or (b) synthesize the anatomical structure and the physiology of blood flow and perfusion of the sample (160) in terms of a spatial blood flow rate distribution, wherein the imaging system is directed and focused on a field of view (FOV) in a region of interest of the sample using an endoscope, and wherein the blood flow rate distribution is based on a relation between velocity distribution of the velocity vector u in m/sec in the region of interest or field of view (FOV) and the blood flow distribution.

2. The system of Claim 1, wherein the endoscope is configured to provide the information related to the first, second and at least third light sources (130, 131, 132) to the processor and is included in an endoscopic imaging system, the endoscopic imaging system comprising:

the multi-sensor camera (110);

a control unit configured to control the multi-sensor camera (110) and process images associated therewith;

an image management unit configured to display and manipulate the images associated with the multi-sensor camera (110); and

a system interface configured to translate the information related to the first, second and at least third light source (130, 131, 132) to perform within the endoscopic imaging system such that the images associated therewith are displayed and manipulated.

3. The system of Claim 2, wherein the endoscopic imaging system comprises at least one of:

a system for endoscopic surgery including at least one of laparoscopy, thorascoscopy, and cystoscopy;

a system for minimally invasive surgical procedures using the endoscope for illumination, visualizing, and manipulation; and

a system for robotic procedures using the endoscope for illumination, visualizing, and manipulation.

4. The system of Claim 2, wherein the endoscopic imaging system is configured:

to illuminate cavity tissues and organs during a procedure;

to visualize cavity tissues and organs for surgical intervention; and/or

for surgical manipulation of cavity tissues and organs for surgical intervention.

5. The system of Claim 2, wherein the endoscopic imaging system is configured to illuminate the region of interest using fibers, fiber compositions, fiber arrangements, lenses, diffusers, collimators, and/or expanders.

6. The system of Claim 2:

wherein the multi-sensor camera (110) and is configured to have speeds, focus, accuracy and fidelity to obtain images using the endoscopic imaging system; and

wherein the multi-sensor camera is coupled to optic fibers of the endoscope.

7. The system of Claim 6, wherein the plurality of sensors of the multi-sensor camera (110) are arranged along a continuum from an endoscope tip to a distance remote from the endoscope coupled by image capture cabling.

8. The system of Claim 2, wherein the endoscope is one of a rigid endoscope, semi-rigid and a flexible endoscope.

9. The system of Claim 8, wherein the endoscope is one of a rigid endoscope having a flexible, navigable tip and a flexible endoscope having a flexible insertion tube.

10. The system of Claim 1, wherein the light at the at least the third wavelength is configured to assess a specific physiologic parameter.

11. The system of Claim 1, wherein the system comprises one or more of a hand-held system, a finger probe unit, a skin patch and a mobile system.

**12.** The system of Claim 1, wherein the multispectral imaging system comprises different blood flow and perfusion measurement technologies in one system, wherein the blood flow and perfusion measurement technologies comprise one or more of laser speckle imaging (LSI), laser Doppler imaging (LDI), florescence imaging and reflectance imaging.

**13.** A non invasive method for multispectral imaging in a multispectral imaging system, excluding endoscopic procedures that include manipulation of tissue, the method comprising:

imaging a sample (160) using a first light source (130) having a first wavelength;
imaging the sample (160) using a second light source (131), different from the first light source (130), having a second wavelength, different from the first wavelength;
receiving information related to the first and second light sources (130, 131) from the sample at a multi-sensor camera (110) having a plurality of sensors, wherein light at the first wavelength is configured to reflect off a surface of the sample (160) into a first of the plurality of sensors of the multi-sensor camera (110) and light at the second wavelength is configured to penetrate the sample (160) and provide information related to the sample to a second of the plurality of sensors of the multi-sensor camera (110), the light at the first and second wavelengths being aligned and synchronized when provided to the first and second sensors of the multi-sensor camera (110); and combining the information related to the first and second light sources received by the multi-sensor camera (110) using at least one processor to (a) image an anatomical structure of the sample and image physiology of blood flow and perfusion of the sample (160), and/or (b) synthesize the anatomical structure and the physiology of blood flow and perfusion of the sample (160) in terms of a spatial blood flow rate distribution, wherein the blood flow rate distribution is based on a relation between velocity distribution of the velocity vector u in m/sec in the region of interest or field of view (FOV) and the blood flow distribution.

**14.** The method of Claim 13:
wherein illumination, analysis and display is performed using the multispectral imaging system; and wherein the multi-spectral imaging system is configured to communicate with a multi-spectral
physiologic visualization (MSPV) operating system.

**Patentansprüche**

**1.** Multispektrales Bildgebungssystem, wobei das System umfasst:

eine erste Lichtquelle (130), welche eine erste Wellenlänge aufweist, die dazu eingerichtet ist, eine Probe (160) abzubilden;
eine von der ersten Lichtquelle verschiedene zweite Lichtquelle (131), welche eine von der ersten Wellenlänge verschiedene zweite Wellenlänge aufweist, die dazu eingerichtet ist, die Probe (160) abzubilden;
wenigstens eine von der ersten und der zweiten Lichtquelle verschiedene dritte Lichtquelle (132), welche eine von der ersten und der zweiten Wellenlänge verschiedene dritte Wellenlänge aufweist, die dazu eingerichtet ist, die Probe (160) abzubilden;
eine Multisensor-Kamera (110), welche eine Mehrzahl von Sensoren aufweist, wobei jeder der Sensoren dazu eingerichtet ist, zu einer gleichen Zeit Informationen in Bezug auf eine der ersten, der zweiten und der wenigstens einen dritten Lichtquelle von der Probe (160) zu empfangen, wobei Licht mit der ersten Wellenlänge dazu eingerichtet ist, eine Fläche der Probe (160) in einen ersten der Mehrzahl von Sensoren der Multisensor-Kamera (110) abzubilden; wobei Licht mit der zweiten Wellenlänge dazu eingerichtet ist, die Probe (160) bis zu einer ersten Tiefe zu durchdringen und einem zweiten der Mehrzahl von Sensoren der Multisensor-Kamera (110) Informationen in Bezug auf die Probe (160) bereitzustellen; und wobei Licht mit der wenigstens einen dritten Wellenlänge dazu eingerichtet ist, die Probe (160) bis zu einer von der ersten Tiefe des Lichts mit der zweiten Wellenlänge verschiedenen zweiten Tiefe zu durchdringen und einem dritten der Mehrzahl von Sensoren der Multisensor-Kamera (110) Informationen in Bezug auf die Probe (160) bereitzustellen, wobei die Informationen in Bezug auf die erste, die zweite und die dritte Lichtquelle ausgerichtet und synchronisiert sind, wenn sie dem ersten, dem zweiten und dem dritten Sensor der Multisensor-Kamera (110) bereitgestellt werden; und
einen Prozessor, welcher dazu eingerichtet ist, die Informationen in Bezug auf die erste, die zweite und die wenigstens eine dritte Lichtquelle, welche durch die Multisensor-Kamera (110) bereitgestellt sind, zu kombinieren, um (a) eine anatomische Struktur der Probe (160) abzubilden und eine Physiologie eines Blutflusses und einer Durchblutung der Probe (160) abzubilden und/oder (b) die anatomische Struktur und die Physiologie eines Blutflusses und einer Durchblutung der Probe (160) in Hinblick auf eine räumliche Blutflussratenverteilung zu synthetisieren,

wobei das Bildgebungssystem unter Verwendung eines Endoskops auf ein Sichtfeld (FOV) in einem Bereich von Interesse der Probe gerichtet und fokussiert ist und wobei die Blutflussratenverteilung auf einer Beziehung zwischen einer Geschwindigkeitsverteilung des Geschwindigkeitsvektors u in m/s in dem Bereich von Interesse oder einem Sichtfeld (FOV) und der Blutflussverteilung basiert.

2. System nach Anspruch 1, wobei das Endoskop dazu eingerichtet ist, dem Prozessor die Informationen in Bezug auf die erste, die zweite und die wenigstens eine dritte Lichtquelle (130, 131, 132) bereitzustellen, und in einem endoskopischen Bildgebungssystem umfasst ist, wobei das endoskopische Bildgebungssystem umfasst:

die Multisensor-Kamera (110);

eine Steuereinheit, welche dazu eingerichtet ist, die Multisensor-Kamera (110) zu steuern und damit assoziierte Bilder zu verarbeiten;

eine Bildverwaltungseinheit, welche dazu eingerichtet ist, die mit der Multisensor-Kamera (110) assoziierten Bilder anzuzeigen und zu manipulieren; und

eine Systemschnittstelle, welche dazu eingerichtet ist, die Informationen in Bezug auf die erste, die zweite und die wenigstens eine dritte Lichtquelle (130, 131, 132) derart zu übersetzen, dass sie innerhalb des endoskopischen Bildgebungssystems ausgeführt werden, so dass die damit assoziierten Bilder angezeigt und manipuliert werden.

3. System nach Anspruch 2, wobei das endoskopische Bildgebungssystem wenigstens eines umfasst, aus:

einem System für endoskopische Chirurgie, umfassend wenigstens eines aus Laparoskopie, Thorakoskopie und Zystoskopie;

einem System für minimalinvasive chirurgische Eingriffe unter Verwendung des Endoskops zur Beleuchtung, Visualisierung und Manipulation; und

einem System für robotergestützte Eingriffe unter Verwendung des Endoskops zur Beleuchtung, Visualisierung und Manipulation.

4. System nach Anspruch 2, wobei das endoskopische Bildgebungssystem eingerichtet ist:

um Hohlraumgewebe und Organe während eines Eingriffs zu beleuchten;

um Hohlraumgewebe und Organe für einen chirurgischen Eingriff zu beleuchten; und/oder

für eine chirurgische Manipulation von Hohlraumgewebe und Organen für einen chirurgischen Eingriff.

5. System nach Anspruch 2, wobei das endoskopische Bildgebungssystem dazu eingerichtet ist, unter Verwendung von Fasern, Faserzusammensetzungen, Faseranordnungen, Linsen, Diffusoren, Kollimatoren und/oder Expandern den Bereich von Interesse zu beleuchten.

6. System nach Anspruch 2:

wobei die Multisensor-Kamera (110) derart eingerichtet ist, dass sie Geschwindigkeiten, Fokus, Genauigkeit und Wiedergabetreue aufweist, um unter Verwendung des endoskopischen Bildgebungssystems Bilder zu erhalten; und

wobei die Multisensor-Kamera mit optischen Fasern des Endoskops gekoppelt ist.

7. System nach Anspruch 6, wobei die Mehrzahl von Sensoren der Multisensor-Kamera (110) von einer Endoskopspitze entlang eines Kontinuums zu einem von dem Endoskop entfernten Abstand angeordnet ist, gekoppelt durch Bildaufnahmekabel.

8. System nach Anspruch 2, wobei das Endoskop eines ist aus einem starren Endoskop, einem halbstarren Endoskop und einem flexiblen Endoskop.

9. System nach Anspruch 8, wobei das Endoskop eines ist aus einem starren Endoskop, welches eine flexible, navigierbare Spitze aufweist, und einem flexiblen Endoskop, welches ein flexibles Einführrohr aufweist.

10. System nach Anspruch 1, wobei das Licht mit der wenigstens einen dritten Wellenlänge dazu eingerichtet ist, einen spezifischen physiologischen Parameter zu beurteilen.

**11.** System nach Anspruch 1, wobei das System eines oder mehrere aus einem Handheld-System, einer Fingersonden-Einheit, einem Hautpflaster und einem mobilen System umfasst.

**12.** System nach Anspruch 1, wobei das multispektrale Bildgebungssystem verschiedene Blutfluss- und Durchblutungs-Messtechnologien in einem System umfasst, wobei die Blutfluss- und Durchblutungs-Messtechnologien eines oder mehrere aus Laserspektralbildgebung (LSI), Laserdopplerbildgebung (LDI), Fluoreszenzbildgebung und Reflexionsbildgebung umfassen.

**13.** Nicht-invasives Verfahren für eine multispektrale Bildgebung in einem multispektralen Bildgebungssystem, ausgenommen endoskopische Eingriffe, welche eine Manipulation von Gewebe umfassen, wobei das Verfahren umfasst:

Abbilden einer Probe (160) unter Verwendung einer ersten Lichtquelle (130), welche eine erste Wellenlänge aufweist;

Abbilden der Probe (160) unter Verwendung einer von der ersten Lichtquelle (130) verschiedenen zweiten Lichtquelle (131), welche eine von der ersten Wellenlänge verschiedene zweite Wellenlänge aufweist;

Empfangen von Informationen in Bezug auf die erste und die zweite Lichtquelle (130, 131) von der Probe an einer Multisensor-Kamera (110), welche eine Mehrzahl von Sensoren aufweist, wobei Licht mit der ersten Wellenlänge dazu eingerichtet ist, von einer Fläche der Probe (160) in einen ersten der Mehrzahl von Sensoren der Multisensor-Kamera (110) reflektiert zu werden, und Licht mit der zweiten Wellenlänge dazu eingerichtet ist, die Probe (160) zu durchdringen und einem zweiten der Mehrzahl von Sensoren der Multisensor-Kamera (110) Informationen in Bezug auf die Probe bereitzustellen, wobei das Licht mit der ersten und der zweiten Wellenlänge ausgerichtet und synchronisiert ist, wenn es dem ersten und dem zweiten Sensor der Multisensor-Kamera (110) bereitgestellt wird; und

Kombinieren der Informationen in Bezug auf die erste und die zweite Lichtquelle, welche von der Multisensor-Kamera (110) empfangen worden sind, unter Verwendung wenigstens eines Prozessors, um (a) eine anatomische Struktur der Probe abzubilden und eine Physiologie eines Blutflusses und einer Durchblutung der Probe (160) abzubilden und/oder (b) die anatomische Struktur und die Physiologie eines Blutflusses und einer Durchblutung der Probe (160) in Hinblick auf eine räumliche Blutflussratenverteilung zu synthetisieren, wobei die Blutflussratenverteilung auf einer Beziehung zwischen einer Geschwindigkeitsverteilung des Geschwindigkeitsvektors u in m/s in dem Bereich von Interesse oder einem Sichtfeld (FOV) und der Blutflussverteilung basiert.

**14.** Verfahren nach Anspruch 13:
wobei eine Beleuchtung, eine Analyse und eine Anzeige unter Verwendung des multispektralen Bildgebungssystems ausgeführt wird; und wobei das multispektrale Bildgebungssystem dazu eingerichtet ist, mit einem Multispektral-Physiologie-Visualisierungs- (MSPV) -Betriebssystem zu kommunizieren.

## Revendications

**1.** Système d'imagerie multispectrale, le système comprenant :

une première source de lumière (130) présentant une première longueur d'onde configurée pour imager un échantillon (160) ;

une deuxième source de lumière (131), différente de la première source de lumière, présentant une deuxième longueur d'onde, différente de la première longueur d'onde, configurée pour imager l'échantillon (160) ;

au moins une troisième source de lumière (132), différente des première et deuxième sources de lumière, présentant une troisième longueur d'onde, différente des première et deuxième longueurs d'onde, configurée pour imager l'échantillon (160) ;

une caméra multi-capteurs (110) présentant une pluralité de capteurs, chacun de la pluralité de capteurs étant configuré pour recevoir en même temps des informations relatives à l'une des première, deuxième et au moins troisième sources de lumière à partir de l'échantillon (160), dans lequel de la lumière à la première longueur d'onde est configurée pour imager une surface de l'échantillon (160) dans un premier de la pluralité de capteurs de la caméra multi-capteurs (110) ; dans lequel de la lumière à la deuxième longueur d'onde est configurée pour pénétrer dans l'échantillon (160) à une première profondeur et fournir des informations relatives à l'échantillon (160) à un deuxième de la pluralité de capteurs de la caméra multi-capteurs (110) ; et dans lequel de la lumière à la au moins troisième longueur d'onde est configurée pour pénétrer dans l'échantillon (160) à une seconde profondeur différente de la première profondeur de la lumière à la deuxième longueur d'onde, et fournir des informations relatives à l'échantillon (160) à un troisième de la pluralité de capteurs de la caméra multi-capteurs (110), les informations relatives aux première, deuxième et troisième sources de lumière étant alignées et synchronisées lorsqu'elles sont fournies aux premier, deuxième et troisième capteurs de la caméra multi-capteurs (110) ; et

un processeur configuré pour combiner les informations relatives à la première, à la deuxième et à la au moins troisième source de lumière fournies par la caméra multi-capteurs (110) pour (a) imager une structure anatomique de l'échantillon (160) et une physiologie d'image de flux sanguin et de perfusion de l'échantillon (160), et/ou (b) synthétiser la structure anatomique et la physiologie du flux sanguin et de la perfusion de l'échantillon (160) en termes de distribution spatiale du débit d'écoulement sanguin,

dans lequel le système d'imagerie est dirigé et focalisé sur un champ de vision (FOV) dans une région d'intérêt de l'échantillon en utilisant un endoscope, et dans lequel la distribution de débit d'écoulement sanguin est basée sur une relation entre une distribution de vitesse du vecteur de vitesse u en m/sec dans la région d'intérêt ou le champ de vision (FOV) et la distribution de flux sanguin.

2. Système selon la revendication 1, dans lequel l'endoscope est configuré pour fournir les informations relatives à la première, à la deuxième et à la au moins une troisième sources de lumière (130, 131, 132) au processeur et est inclus dans un système d'imagerie endoscopique, le système d'imagerie endoscopique comprenant :

la caméra multi-capteurs (110) ;

une unité de commande configurée pour commander la caméra multi-capteurs (110) et traiter des images qui lui sont associées ;

une unité de gestion d'images configurée pour afficher et manipuler les images associées à la caméra multi-capteurs (110) ; et

une interface système configurée pour translater les informations relatives à la première, la deuxième et à la au moins une troisième source de lumière (130, 131, 132) pour mise en œuvre dans le système d'imagerie endoscopique de telle sorte que les images qui lui sont associées soient affichées et manipulées.

3. Système selon la revendication 2, dans lequel le système d'imagerie endoscopique comprend au moins un parmi :

un système de chirurgie endoscopique incluant au moins une parmi une laparoscopie, une thorascoscopie et une cystoscopie ;

un système pour des procédures chirurgicales invasives au minimum utilisant l'endoscope pour l'éclairage, la visualisation et la manipulation ; et

un système pour des procédures robotiques utilisant l'endoscope pour l'éclairage, la visualisation et la manipulation.

4. Système selon la revendication 2, dans lequel le système d'imagerie endoscopique est configuré :

pour éclairer les tissus et les organes de la cavité pendant une procédure ;

pour visualiser les tissus et les organes de la cavité pour une intervention chirurgicale ; et/ou

pour la manipulation chirurgicale des tissus et des organes de la cavité pour une intervention chirurgicale.

5. Système selon la revendication 2, dans lequel le système d'imagerie endoscopique est configuré pour éclairer la région d'intérêt en utilisant des fibres, des compositions de fibres, des agencements de fibres, des lentilles, des diffuseurs, des collimateurs et/ou des expanseurs.

6. Système selon la revendication 2 :

dans lequel la caméra multi-capteurs (110) et est configurée pour avoir des vitesses, une mise au point, une précision et une fidélité pour obtenir des images en utilisant le système d'imagerie endoscopique ; et

dans lequel la caméra multi-capteurs est couplée à des fibres optiques de l'endoscope.

7. Système selon la revendication 6, dans lequel la pluralité de capteurs de la caméra multi-capteurs (110) sont agencés le long d'un continuum depuis une pointe d'endoscope jusqu'à une distance éloignée de l'endoscope, couplés par un câblage de capture d'image.

8. Système selon la revendication 2, dans lequel l'endoscope est un parmi un endoscope rigide, un endoscope semi-rigide et un endoscope flexible.

9. Système selon la revendication 8, dans lequel l'endoscope est un parmi un endoscope rigide présentant une pointe flexible et pouvant naviguer et un endoscope flexible présentant un tube d'insertion flexible.

10. Système selon la revendication 1, dans lequel la lumière à la au moins troisième longueur d'onde est configurée pour évaluer un paramètre physiologique spécifique.

11. Système selon la revendication 1, dans lequel le système comprend un ou plusieurs parmi un système portatif, une unité de sonde digitale, un timbre cutané et un système mobile.

12. Système selon la revendication 1, dans lequel le système d'imagerie multispectrale comprend différentes technologies de mesure du flux sanguin et de la perfusion dans un système, dans lequel les technologies de mesure du flux sanguin et de la perfusion comprennent une ou plusieurs parmi l'imagerie par taches laser (LSI), l'imagerie Doppler laser (LDI), l'imagerie par fluorescence et l'imagerie par réflectance.

13. Procédé non invasif d'imagerie multispectrale dans un système d'imagerie multispectrale, à l'exclusion de procédures endoscopiques qui incluent une manipulation de tissus, le procédé comprenant les étapes consistant à :

imager un échantillon (160) en utilisant une première source de lumière (130) présentant une première longueur d'onde ;
imager un échantillon (160) en utilisant une deuxième source de lumière (131), différente de la première source de lumière (130), présentant une deuxième longueur d'onde, différente de la première longueur d'onde ;
recevoir des informations relatives aux première et deuxième sources de lumière (130, 131) à partir de l'échantillon au niveau d'une caméra multi-capteurs (110) présentant une pluralité de capteurs, dans lequel la lumière à la première longueur d'onde est configurée pour se réfléchir sur une surface de l'échantillon (160) dans un premier de la pluralité de capteurs de la caméra multi-capteurs (110) et la lumière à la deuxième longueur d'onde est configurée pour pénétrer dans l'échantillon (160) et fournir des informations relatives à l'échantillon à un deuxième de la pluralité de capteurs de la caméra multi-capteurs (110), les lumières à la première et à la deuxièmes longueur d'onde étant alignées et synchronisées lorsqu'elles sont fournies aux premier et deuxième capteurs de la caméra multi-capteurs (110) ; et
combiner les informations relatives aux première et deuxième sources de lumière reçues par la caméra multi-capteurs (110) en utilisant au moins un processeur pour (a) imager une structure anatomique de l'échantillon et une physiologie d'image du flux sanguin et de la perfusion de l'échantillon (160), et/ou (b) synthétiser la structure anatomique et la physiologie du flux sanguin et de la perfusion de l'échantillon (160) en termes de distribution spatiale du débit d'écoulement sanguin, dans lequel la distribution de débit d'écoulement sanguin est basée sur une relation entre une distribution de vitesse du vecteur de vitesse u en m/sec dans la région d'intérêt ou le champ de vision (FOV) et la distribution de flux sanguin.

14. Procédé selon la revendication 13 :
dans lequel l'éclairage, l'analyse et l'affichage sont effectués en utilisant le système d'imagerie multispectrale ; et dans lequel le système d'imagerie multispectrale est configuré pour communiquer avec un système d'exploitation de visualisation physiologique multispectrale (MSPV).

FIG. 1A

FIG. 1B

FIG. 1C

MULTI-SENSOR CAMERA
110'

FIG. 2

I/O DATA PORTS
346

PROCESSOR
338

MEMORY
336

USER INTERFACE
344

DATA PROCESSING SYSTEM
300

FIG. 3

FIG. 4

FIG. 5A

FIG. 5B

FIG. 6A

FIG. 6B

FIG. 7A

HIGH FLOW

MEDIUM FLOW

LOW FLOW

FIG. 7B

FIG. 8A

HIGH FLOW

MEDIUM FLOW

LOW FLOW

FIG. 8B

PANEL A

PANEL B

FIG. 9A    950

FIG. 9B    950

PANEL A

*FIG. 10A*

PANEL B

*FIG. 10B*

PANEL C

*FIG. 10C*

PANEL D (A+B+C)

*FIG. 10D*

HIGH FLOW

MEDIUM FLOW

LOW FLOW

PANEL A

FIG. 11A

PANEL B

FIG. 11B

PANEL C (A ADJUSTS BRIGHTNESS
AND B ADJUSTS COLOR)

FIG. 11C

EP 3 675 723 B1

PANEL A

FIG. 12A

PANEL B

FIG. 12B

PANEL C

FIG. 12C

PANEL D

FIG. 12D

35

PANEL A

FIG. 13A

PANEL B

FIG. 13B

PANEL C

FIG. 13C

PANEL D

FIG. 13D

FIG. 14A

FIG. 14B

FIG. 14C

FIG. 14D

FIG. 14E

FIG. 15A

FIG. 15B

FIG. 17A

FIG. 17B

FIG. 18A

FIG. 18B

FIG. 19B

FIG. 19A

2057S

EXTENSION ARMS
2057

2057S

HOUSING, CONTROL
BUTTONS, INDICATOR
LIGHT ETC.

OPERATING
HEAD MODULE
2067

SUBJECT
2077

DISPLAY
MONITOR(S)
2047

MOBILE CART
2017

COMPUTER TOWER
2027

CONTROL BOX
2037

WHEELS WITH
BRAKE
2018

FIG. 20

COMPUTER AND
DISPLAY
2128

POWER SUPPLY AND
CONTROL UNIT
2138

2148

2118

FLEXIBLE EXTENSION ARM THAT
PROVIDES FOR STABLE IMAGING
DISTANCE

FIG. 21A

2118

MOVABLE OPERATING
HEAD UNIT

FIG. 21B

COMPUTER AND
DISPLAY
2228

POWER SUPPLY AND
CONTROL UNIT
2238

2248

FINGER PROBE UNIT
2258

PATCH
2268

FIG. 22

PORTABLE HAND-
HELD OPERATING
HEAD UNIT

COMPUTER AND DISPLAY
POWER SUPPLY AND CONTROL UNIT

2373

FIG. 23

FIG. 24

FIG. 25

FIG. 26A

FIG. 26B

FIG. 26C

FIG. 26D

FIG. 27

FIG. 28

TIME MARK (SECONDS) AND IMAGE QUALITY (COLOR) BAR
GREEN: GOOD IMAGE QUALITY
YELLOW: MEDIUM IMAGE QUALITY
RED: POOR IMAGE QUAILTY

FIG. 29A

TIME MARK (SECONDS) AND IMAGE QUALITY (COLOR) BAR
GREEN: GOOD IMAGE QUALITY
YELLOW: MEDIUM IMAGE QUALITY
RED: POOR IMAGE QUAILTY

FIG. 29B

START

IMAGE SAMPLE USING A FIRST LIGHT SOURCE HAVING A FIRST WAVELENGTH THROUGH AN ENDOSCOPIC SYSTEM — 3000

IMAGE SAMPLE USING A SECOND LIGHT SOURCE, DIFFERENT FROM THE FIRST LIGHT SOURCE, HAVING A SECOND WAVELENGTH THROUGH THE ENDOSCOPIC SYSTEM — 3010

RECEIVE INFOMATION RELATED TO THE FIRST AND SECOND LIGHT SOURCES FROM THE SAMPLE AT A CAMERA — 3020

COMBINE THE INFORMATION RELATED TO THE FIRST AND SECOND LIGHT SOURCES TO PROVIDE BLOOD FLOW AND PERFUSION DATA — 3030

END

*FIG. 30*

FIG. 31

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10390718 B2
- US 2013012794 A1
- US 2006241495 A1
- US 2008132794 A1
- WO 2016153741 A1
- US 2017135555 A1